# EUROPÄISCHE PATENTSCHRIFT

(11) **EP 1 012 303 B1**
(45) Veröffentlichungstag und Bekanntmachung des Hinweises auf die Patenterteilung: **27.09.2006**
(21) Anmeldenummer: 98905134.7
(22) Anmeldetag: 27.02.1998
(51) Int. Cl.: C12N 15/57, C12N 9/64, A61K 38/48

(54) **FAKTOR X-DELETIONSMUTANTEN UND ANALOGE DAVON**
FACTOR X DELETION MUTANTS AND ANALOGUES THEREOF
MUTANTS DU FACTEUR X A DELETION D'ACIDES AMINES ET ANALOGUES DESDITS MUTANTS

(30) Priorität: 27.02.1997 AT 33697
(43) Veröffentlichungstag der Anmeldung: 28.06.2000
(73) Patentinhaber: Baxter Aktiengesellschaft, 1221 Wien (AT)
(72) Erfinder: HIMMELSPACH, Michele, 2562 Port (CH); PFLEIDERER, Michael, D-64367 Muehltal (DE); FALKNER, Falko-Günter, A-2304 Orth/Donau (AT); EIBL, Johann, A-1180 Wien (AT); DORNER, Friedrich, A-1230 Wien (AT); SCHLOKAT, Uwe, A-2304 Orth/Donau (AT)
(74) Vertreter: Alge, Daniel
(86) Internationale Anmeldenummer: PCT/AT1998/000046
(87) Internationale Veröffentlichungsnummer: WO 1998/038318

(56) Entgegenhaltungen:
- WOLF D L ET AL: "Design of constructs for the expression of biologically active recombinant human factors X and Xa. Kinetic analysis of the expressed proteins." JOURNAL OF BIOLOGICAL CHEMISTRY., Bd. 266, Nr. 21, 25.Juli 1991, MD US, Seiten 13726-13730, XP002065182 in der Anmeldung erwähnt
- LEYTUS S ET AL: "Gene for human factor X: a blood coagulation factor whose gene organizytion is essentially identical with that of factor IX and protein c" BIOCHEMISTRY, Bd. 25, Nr. 18, September 1986, Seiten 5098-5102, XP002065183 in der Anmeldung erwähnt

## Beschreibung

Die Erfindung betrifft Faktor XΔ-Analoge mit einer Deletion der Aminosäuren Arg180 bis Arg234 und eine Modifikation im Bereich der Aminosäuresequenz zwischen Gly173 bis Arg179, Präparationen enthaltend die erfindungsgemäße Faktor XΔ-Analoge oder Faktor Xa-Analoge, sowie Verfahren zur Herstellung der erfindungsgemäßen Faktor XΔ-Analogen.

Nach Initiierung des Blutgerinnungsprozesses verläuft die Gerinnungskaskade durch die sequentielle Aktivierung von verschiedenen Proenzymen (Zymogenen) im Blut in ihre aktiven Formen, den Serinproteasen. Dazu gehören u.a. Faktor XII/XIIa, Faktor XI/XIa, Faktor IX/IXa, Faktor X/Xa, Faktor VII/VIIa und Prothrombin/Thrombin. Die meisten dieser Enzyme sind im physiologischen Zustand nur aktiv, wenn sie in einem Komplex an einer Membranoberfläche assoziiert sind. Ca-Ionen sind in viele dieser Prozesse involviert. Die Blutgerinnung folgt entweder dem intrinsischen Weg, bei dem alle Proteinkomponenten im Blut vorhanden sind, oder dem extrinsischen Weg, bei dem der Gewebefaktor eine kritische Rolle spielt. Der Wundverschluß erfolgt schließlich durch die Spaltung von Fibrinogen zu Fibrin durch Thrombin.

Der Prothrombinase-Komplex ist verantwortlich für die Aktivierung von Prothrombin zu Thrombin. Thrombin ist ein wichtiges Enzym, das sowohl als Prokoagulant als auch als Antikoagulant wirken kann. Der Prothrombinase-Komplex, an dem u.a. Faktor Va (als Cofaktor) und Faktor Xa (als Serinprotease) beteiligt sind, assembliert in einer Ca-abhängigen Assoziation an der Oberfläche von Phospholipiden. Es wird diskutiert, daß dabei die katalytische Komponente des Prothrombinase-Komplexes Faktor Xa ist.

Faktor X (Stuart/Prower-Faktor) ist ein Vitamin K-abhängiges Koagulationsglykoprotein, das durch die intrinsische und extrinsische Blutgerinnungskaskade aktiviert werden kann. Das primäre Translationsprodukt von Faktor X (pre-pro-FX) besitzt 488 Aminosäuren und wird von der Leber oder humanen Hepatoma-Zellen zunächst als einzelkettiges 75 kD Vorläuferprotein synthetisiert. Im Plasma liegt Faktor X weitgehend als zweikettiges Molekül vor (Fair et al., 1984, Blood 64:194-204).

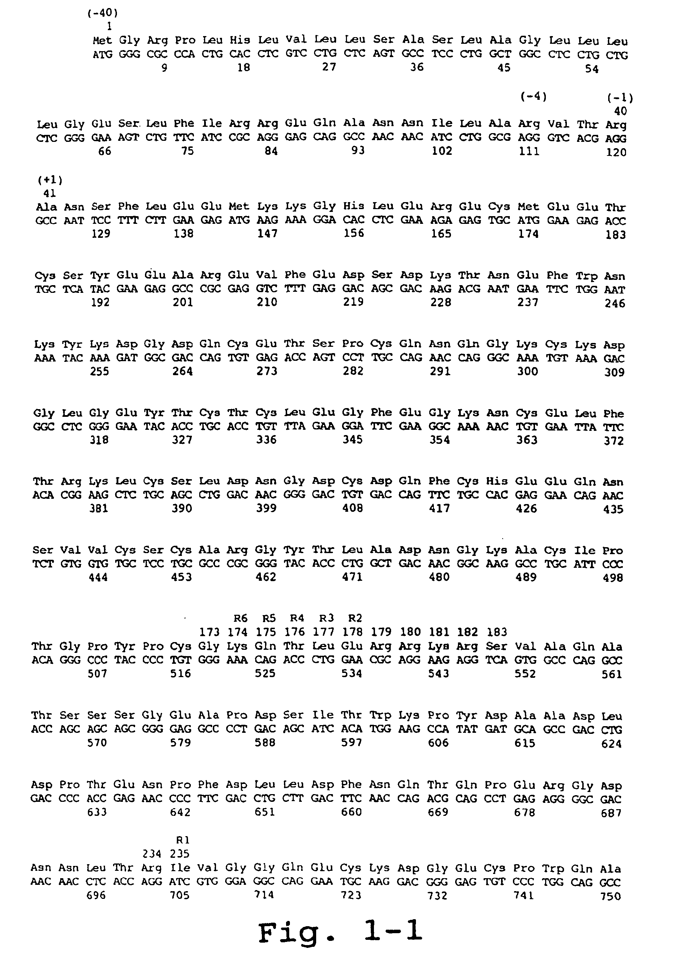

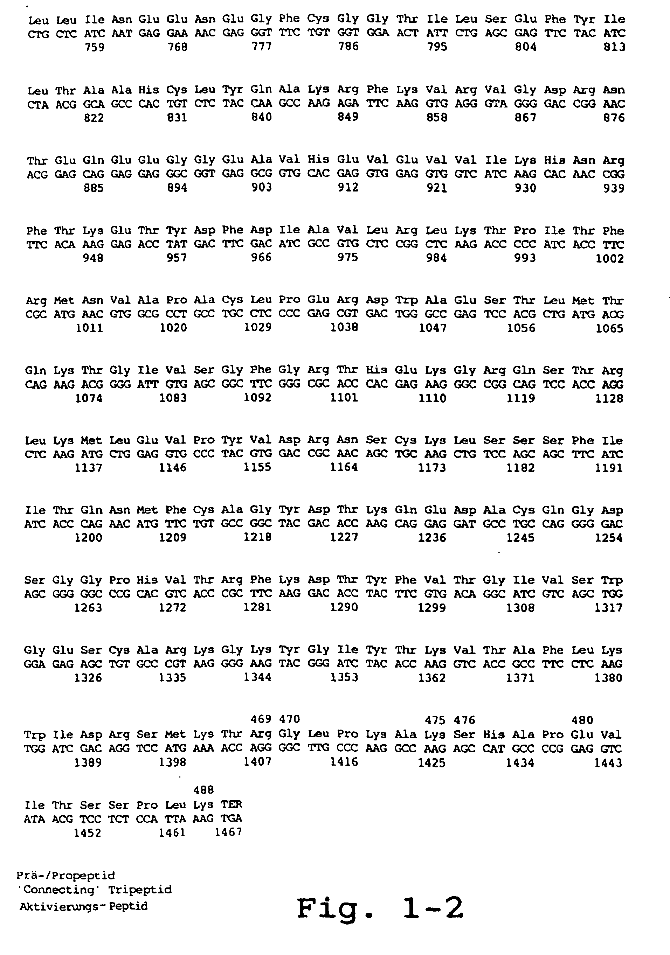

Während der Biosynthese wird nach Abspaltung der pre-Sequenz durch eine Signalpeptidase (zwischen Ser23/Leu24) und des Propeptides (zwischen Arg40/Ala41) das einzelkettige Faktor X-Molekül durch Prozessierung und Enfernung des Tripeptides Arg180-Lys181-Arg-182 in die zweikettige Form, bestehend aus der ca. 22 kD leichten Kette und der ca. 50 kD schweren Kette, die miteinander über eine Disulfid-Brücke verbunden sind, gespalten (Figur 1). Faktor X zirkuliert im Plasma daher als zweikettiges Molekül.

Während des Blutgerinnungsprozesses wird Faktor X vom inaktiven Zymogen zur aktiven Protease Faktor Xa durch limitierte Proteolyse konvertiert, wobei die Aktivierung von Faktor X zu Faktor Xa in einem von 2 membrangebundenen Komplexen erfolgen kann: dem extrinsischen Faktor VIIa/Gewebefaktor-Komplex oder dem intrinsischen Faktor VIIIa-Faktor IXa-Phospholipid-Ca-Komplex oder "Tenase-Komplex" (Mertens et al., 1980, Biochem. J. 185:647-658). Eine proteolytische Spaltung zwischen Aminosäuren Arg 234/Ile 235 führt zur Freisetzung eines 52 Aminosäuren langen Aktivierungspeptids vom N-Terminus der schweren Kette und damit zur Bildung des aktiven Enzyms, Faktor Xa. Das katalytische Zentrum des Faktor Xa ist auf der schweren Kette lokalisiert.

Die Aktivierung über den Faktor VIIa-TF(extrinsischen)-Komplex führt zur Bildung von Faktor Xaα (35 kD) und Faktor Xaβ (31 kD), wobei bei geringen Konzentrationen von Faktor VIIa im Komplex auch ein Polypeptid von 42 (kD) auftritt. Die Bildung von Faktor Xaα erfolgt über eine Spaltung bei Arg234/Ile 235 der schweren Kette und repräsentiert die Aktivierung von Faktor X zu Faktor Xa. Das Auftreten von Faktor Xaβ resultiert vermutlich aus einer autokatalytischen Spaltung bei Arg469/Gly470 im C-Terminus der schweren Kette von Faktor Xaα und der Abspaltung eines 4.5 kD-Peptides. Faktor Xaβ besitzt ebenfalls wie Faktor Xaα katalytische Aktivität. Es wurde jedoch gezeigt, daß durch die Spaltung von Faktor Xaα zu Faktor Xaβ eine Plasminogen-Rezeptorbindungsstelle entsteht und Faktor Xaβ gegebenenfalls fibrinolytische Aktivität aufweist bzw. an Fibrinolyse als Cofaktor beteiligt ist. Die Umwandlung von Faktor Xaα zu Faktor Xaβ ist jedoch langsamer als die Bildung von Thrombin, wodurch eine Initiierung der Fibrinolyse vor Ausbildung eines Blutklots verhindert wird (Pryzdial et al., 1996, J. Biol. Chem. 271:16614-16620; Pryzdial et al., 1996, J. Biol. Chem. 271:16621-16626).

Das 42 kD-Polypeptid resultiert aus einer Prozessierung im C-Terminus der schweren Kette zwischen Arg469/Gly470 ohne vorherige Prozessierung zwischen Arg234/Ile 235. Dieses Intermediat besitzt ebenso wie ein Faktor Xaγ-Fragment, das durch Proteolyse bei Lys370 entsteht, keine katalytische Aktivität (Mertens et al., 1980, Biochem. J. 185:647-658; Pryzdial et al., 1996, J. Biol. Chem. 271:16614-16620).

Die Aktivierung von Faktor X im intrinsischen Weg wird katalysiert durch den Faktor IXa-Faktor VIIIa-Komplex. Während der Aktivierung werden die gleichen Prozessierungsprodukte erhalten, jedoch wird das Faktor Xaβ-Produkt in einem höheren Ausmaß erhalten als andere Faktor X-Prozessierungsprodukte (Jesty et al., 1974, J. Biol. Chem. 249:5614).

In vitro kann Faktor X beispielsweise durch Russell's Viper Venom (RVV) oder Trypsin (Bajaj et al., 1973, J.Biol. Chem. 248:7729-7741) oder gereinigte physiologische Aktivatoren, wie FVIIa/TF-Komplex oder Faktor IXa/Faktor VIIIa-Komplex aktiviert werden (Mertens et al., 1980, Biochem. J. 185:647-658).

Kommerziell erhältliche Faktor X-Produkte aus Plasma enthalten zumeist eine Mischung aus Faktor Xaα und Faktor Xaβ, da nach Aktivierung von Faktor X zu Faktor Xa in erster Linie Faktor Xaα entsteht, der in einem autokatalytischen Prozeß wiederum zu Faktor Xaβ gespalten wird.

Um ein einheitliches Faktor Xa-Produkt mit hoher molekularer Integrität herzustellen, wurde in der EP 0 651 054 vorgeschlagen, Faktor X mit RVV über einen längeren Zeitraum zu aktivieren, sodaß das resultierende Endprodukt im wesentlichen Faktor Xaβ enthielt. Sowohl die Nebenprodukte, beispielsweise Faktor Xaα, als auch die Protease wurden anschließend durch mehrere chromatographische Schritte entfernt.

Die cDNA für Faktor X wurde isoliert und charakterisiert (Leytus et al., 1984, Proc. Natl. Acad. Sci., USA, 82:3699-3702; Fung et al., 1985, Proc. Natl. Acad. Sci., USA, 82:3591-3595). Humaner Faktor X wurde in vitro in verschiedenen Zelltypen, wie humanen embryonalen Nierenzellen oder CHO-Zellen exprimiert (Rudolph et al., 1997, Prot. Expr. Purif.10: 373-378, Wolf et al., 1991, J. Biol.Chem. 266:13726-13730). Es wurde jedoch festgestellt, daß bei der rekombinanten Expression von humanem Faktor X die Prozessierung an Position Arg40/Ala41 im Gegensatz zur in vivo-Situation ineffizient erfolgt und unterschiedliche N-Termini an der leichten Kette von Faktor X entstehen (Wolf et al., 1991, J. Biol.Chem. 266:13726-13730). Rekombinanter Faktor X (rFX) wurde durch RVV in vitro zu rFaktor Xa (rFXa) aktiviert oder rFXa direkt exprimiert, wobei das Aktivierungspeptid von Aminosäure 183 bis Aminosäure 234 deletiert und durch ein Tripeptid ersetzt wurde, um eine Prozessierung unmittelbar in eine zweikettige rFXa-Form zu ermöglichen. Gereinigter rFX wurde zu etwa 70% in leichte und schwere Kette prozessiert, während die verbleibenden 30% einzelkettigen rFX mit 75 kD darstellten. Direkte Expression von rFXa führte zwar zur Bildung von aktivem Faktor Xa, jedoch auch zu inaktiven Intermediaten. Wolf et al. (1991, J. Biol. Chem. 266:13726-13730) stellten weiterhin eine verringerte Aktivität von rekombinantem Faktor X fest, die sie auf die schlechtere Aktivierbarkeit des rFX durch RVV sowie auf die inaktive Population an Proteinen und Polypeptiden des einzelkettigen Vorläufermoleküls zurückführten. Insbesondere fanden sie eine hohe Instabilität von rFXa bei Expression durch rekombinante Zellen, was sie auf die hohe Autoproteolyserate zurückführten.

Um die Funktion des C-terminalen Peptides von Faktor Xaα zu untersuchen, führten Eby et al. (1992, Blood 80 (Suppl. 1): 1214 A) ein Stopcodon an Position Gly430 der Faktor X-Sequenz ein. Sie fanden jedoch keinen Unterschied zwischen der Aktivierungsrate von Faktor Xa (FXaα) mit β-Peptid oder einer Deletionsmutante ohne β-Peptid (FXaβ).

Faktor Xa ist ein wichtiger Bestandteil des Prothrombinase-Komplexes und wird daher als primärer Mediator der schnellen Stillung von Blutungen diskutiert, wodurch er geeignet zur Behandlung von Patienten mit Störungen der Blutgerinnung, beispielsweise bei Hämophilie, erscheint.

Insbesondere die Behandlung von Hämophilie-Patienten mit Faktor VIII- oder Faktor IX-Defizienz mit Faktoren-Konzentraten, hergestellt aus Plasma, wird bei längeren Therapiezeiten oft dadurch kompliziert, daß inhibitorische Antikörper gegen diese Faktoren gebildet werden. Es wurden daher eine Reihe von Alternativen entwickelt, um Hämophilie-Patienten mit Faktoren mit einer By-pass-Aktivität zu behandeln. So wurde die Verwendung von Prothrombin-Komplex-Konzentrat, partiell aktiviertem Prothrombinase-Komplex (APPC), Faktor VIIa oder FEIBA vorgeschlagen. Kommerzielle Präparate mit Faktor VIII-Bypass-Aktivität (FEIBA) sind beispielsweise FEIBA® oder Autoplex® . FEIBA etwa enthält vergleichbare Einheiten an Faktor II, Faktor VII, Faktor IX, Faktor X und FEIBA, geringe Mengen an Faktor VIII und Faktor V, sowie Spuren von aktivierten Koagulationsfaktoren, wie Thrombin und Faktor Xa bzw. einen Faktor mit Faktor X-ähnlicher Aktivität (Elsinger, 1982, Activated Prothrombin Complex Concentrates. Ed. Mariani, Russo, Mandelli, p.77-87). Elsinger weist insbesondere auf die Bedeutung einer "Faktor Xa-like"-Aktivität in FEIBA hin. Faktor VIII-Bypass-Aktivität wurde von Giles et al. (1988, British J. Haematology 9:491-497) für eine Kombination von gereinigtem Faktor Xa und Phospholipiden im Tiermodell gezeigt.

Es besteht daher ein großer Bedarf und eine Reihe von verschiedenen Anwendungsgebieten für Faktor X/Xa oder Faktor X/Xa-ähnlichen Proteinen entweder allein oder als Bestandteil eines Koagulationskomplexes in der Blutstillungstherapie.

Die Halbwertszeit von Faktor Xa ist gegenüber dem Zymogen sowohl in vivo als auch in vitro stark herabgesetzt. So kann etwa Faktor X in Glycerin 18 Monate stabil aufbewahrt werden, während Faktor Xa unter gleichen Bedingungen nur 5 Monate stabil ist (Bajaj et al., 1973, J. Biol. Chem. 248:7729-2241) bzw. in Glycerin bei 4°C nach 8 Monaten eine Reduktion der Aktivität um mehr als 60% zeigt (Teng et al., 1981, Thrombosis Res. 22: 213-220). Die Halbwertszeit von Faktor Xa beträgt lediglich 30 Sekunden im Serum.

Aufgrund der Instabilität von Faktor Xa wurde vorgeschlagen, Faktor X-Präparate zu verabreichen (US 4,501,731). Bei lebensbedrohenden Blutungen, insbesondere bei Hämophilie-Patienten, ist jedoch ein Verabreichung von Faktor X wirkungslos, da durch das Fehlen des funktionellen "Tenase-Komplexes" im intrinsischen Blutgerinnungsweg keine ausreichende Aktivierung von Faktor X zu Faktor Xa erfolgen kann und die Aktivierung über den extrinsischen Weg oftmals zu langsam erfolgt, um eine rasche Wirkung zu erzielen. Zudem ist bei Hämophilie-Patienten ausreichend Faktor X vorhanden, der jedoch im Vergleich zu Faktor Xa eine 1000fach geringere Prothrombinase-Aktivität besitzt. In solchen Fällen ist es erforderlich, aktivierten Faktor Xa direkt, gegebenenfalls zusammen mit Phospholipiden, wie bei Giles et al. (1988, British J. Haematology 9:491-497) beschrieben oder mit anderen Koagulationsfaktoren, etwa mit Faktor VIII By-pass-Aktivität, zu verabreichen.

Bei der Herstellung von Faktor Xa aus Faktor X erfolgte die Aktivierung bisher zumeist über unphysiologische Aktivatoren tierischen Ursprungs, wie RVV oder Trypsin, wobei jedoch absolut sichergestellt sein mußte, daß das Endprodukt vollständig frei von diesen Proteasen ist. Wie oben schon erwähnt, werden bei der Aktivierung von Faktor X zu Faktor Xa eine Vielzahl teilweise auch inaktiver Intermediate gebildet (Bajaj et al., 1973, J. Bio. Chem. 248:7729-7741, Mertens et al., 1980, Biochem. J. 185: 647-658). Die Anwesenheit solcher Intermediate führt zu einer Erniedrigung der spezifischen Aktivität des Produktes und gegebenenfalls auch zu solchen Intermediaten, die als Antagonisten der aktiven Serinprotease fungieren können. Zur Herstellung eines einheitlichen, reinen Produktes mit hoher spezifischer Aktivität sind daher bei konventionellen Methoden aufwendige Verfahren zur Aktivierung sowie zur chromatographischen Reinigung erforderlich.

Aufgabe der vorliegenden Erfindung ist es daher eine Präparation zur Verfügung zu stellen, die ein Polypeptid mit Faktor X/Xa-Aktivität enthält, das eine hohe Stabilität aufweist und das ohne die Verwendung einer der üblichen Proteasen, insbesondere tierischen Ursprungs, wie beispielsweise RVV oder Trypsin, zu Faktor Xa aktiviert werden kann. Ein weiteres Ziel ist es, eine pharmazeutische Präparation mit Faktor VIII-Bypass-Aktivität bereitzustellen.

Die Aufgabe wird erfindungsgemäß dadurch gelöst, daß ein Faktor X-Analogon zur Verfügung gestellt wird, das eine Deletion der Aminosäuren Arg180 bis Arg234 der Faktor X-Aminosäuresequenz aufweist sowie eine Modifikation dieser Faktor X-Deletionsmutante im Bereich der Aminosäuresequenz zwischen Gly173 und Arg179. Durch die Deletion der Aminosäuresequenz von Arg180 bis Arg234 werden sowohl das Tripeptid Arg180 bis Arg182 als auch das Aktivierungspeptid Ser183 bis Arg234 deletiert und es entsteht eine direkte Fusion zwischen leichter und schwere Kette von Faktor X und den Aminosäuren Arg179 und Ile235. Diese Fusionssequenz enthält jedoch keine natürlich vorkommende Spaltstelle für eine Protease. Durch Modifikation des Bereiches der Faktor X-Sequenz zwischen Aminosäure Gly173 und Arg179 und gegebenenfalls von Ile235 wird eine erfindungsgemäße Faktor X-Deletionsmutante erhalten, die eine neue, nicht an dieser Position im Polypeptid vorkommende Erkennungs- bzw. Prozessierungsstelle für eine Protease, die das Polypeptid normalerweise nicht an dieser Stelle spaltet, aufweist. Die Modifikation ist dabei mindestens ein Austausch mindestens einer Aminosäure zwischen Position Gly173 und Arg179 und gegebenenfalls von Ile235 der Faktor X-Aminosäuresequenz. Die Position der Aminosäuren ist dabei bezogen auf die Numerierung gemäß der in Fig. 1 dargestellten Sequenz, beginnend mit Met1 und endend mit Lys488. Für die erfindungsgemäße modifizierte Faktor X-Deletionsmutante wird zur Vereinfachung der Nomenklatur die für die komplette Faktor X-Sequenz vorgegebene Aminosäurenumerierung beibehalten, jedoch wird im weiteren die modifizierte Faktor X-Deletionsmutante als Faktor XΔ-Analogon bezeichnet.

Die Modifikation kann dabei eine Substitution mindestens einer Aminosäure oder eine Insertion einer Peptidsequenz, die eine Proteaseerkennungs- bzw. Spaltstelle darstellt, sein. Die Modifikation im erfindungsgemäßen Faktor XΔ-Analogon ist dabei vorzugsweise derart, daß sie für eine Protease aus der Gruppe der Endoproteasen, wie etwa Kexin/Kex2, Furin/PACE, PC1/PC3, PC2, PC4, PACE 4, LPC/PC7 (wie in Barr et al., 1991, Cell 66:1-3 oder in der US 5,460,950 beschrieben), der Serinproteasen, wie etwa Faktor IIa, Faktor VIIa, Faktor IXa, Faktor XIIa, Faktor XIa, Faktor Xa oder von Kallikrein, oder einem Derivat dieser Proteasen, eine Erkennungs- bzw. Spaltsequenz darstellt.

Die Modifikation ist vorzugsweise so ausgewählt, daß die Prozessierung durch eine dieser Proteasen zu einem in seiner biologischen Aktivität dem nativen Faktor Xa entsprechenden Polypeptid führt und Faktor Xa-Aktivität aufweist. Um eine optimale Prozessierung zu erreichen, kann es in einzelnen Fällen notwendig sein, zusätzlich die Aminosäure Ile235 auszutauschen.Vorzugsweise sollte die NH₂-terminale Aminosäure Isoleucin der schweren Kette jedoch nach Aktivierung erhalten bleiben, da Isoleucin einer jener Aminosäuren repräsentiert, denen bei der Bildung der Substratbindungstasche eine wesentliche Funktion zukommt (Watzke et al., 1995, Molecular Basis of Thrombosis and Hemostasis, ed. Katherine High & Harold Roberts). Die erfindungsgemäßen Faktor XΔ-Analoge zeigen einen strukturellen Unterschied, insbesondere auf Aminosäure-Ebene im Vergleich zur nativen Faktor X-Sequenz, besitzen jedoch nach Aktivierung eine vergleichbare Aktivität wie natürlich vorkommender Faktor X bzw. Faktor Xa.

Die Erfindung stellt dabei beispielhaft eine Anzahl von Faktor XΔ-Analogen zur Verfügung, die eine Deletion aufweisen und zusätzlich eine Modifikation zwischen Gly173 und Arg179 und gegebenenfalls von Ile235 aufweisen. Modifikationen können an einer oder mehreren Positionen im Bereich zwischen Aminosäure Gly173 und Arg179, und gegebenenfalls Ile235, bezogen auf die Faktor X-Sequenz mit der Numerierung mit Met1 bis Lys 488 gemäß Fig. 1, sein. Aminosäuresubstitutionen können dabei sein an Position Ile 235 (R1), Arg179, Glu178 (R2), Leu177 (R3), Thr176 (R4), Gln175 (R5) und Lys174 (R6), wobei jedoch vorzugsweise Arg179 unverändert bleibt.

Die erfindungsgemäßen Faktor XΔ-Analogon enthalten vorzugsweise eine Faktor X-Sequenz mit Gly173-R6-R5-R4-R3-R2-Arg179-R1 mit R1= Ile, Val, Ala, Ser oder Thr, R2= Glu, Thr, Pro, Gly, Lys oder Arg; R3= Leu, Phe, Lys, Met, Gln, Ser, Val, Arg oder Pro; R4= Thr, Asn, Asp, Ile, Ser, Pro, Arg oder Lys; R5= Asn, Lys, Ser, Glu, Gln, Ala, His oder Arg und R6= Arg, Asp, Phe, Thr, Leu oder Ser, darstellt.

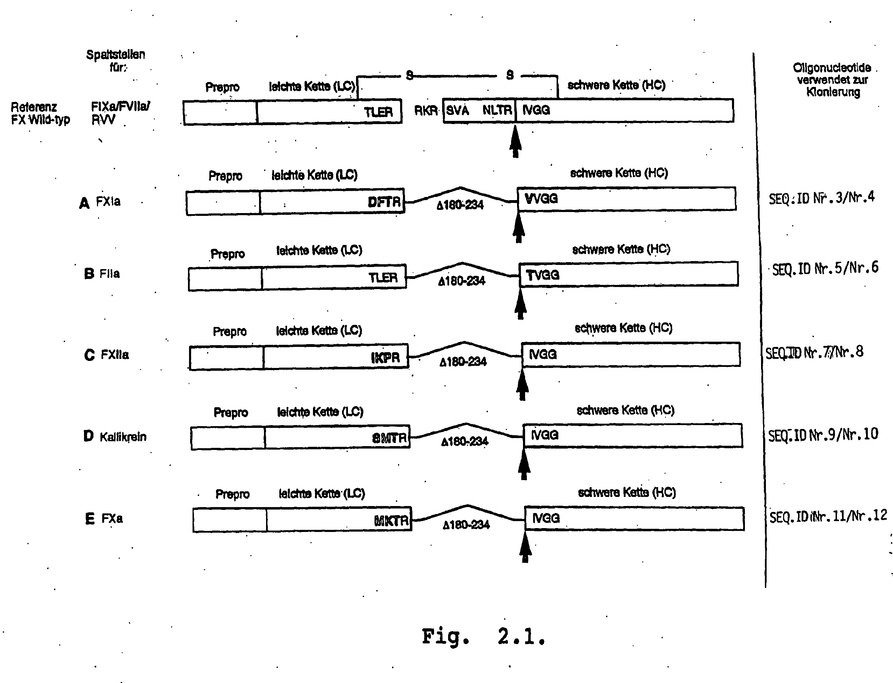

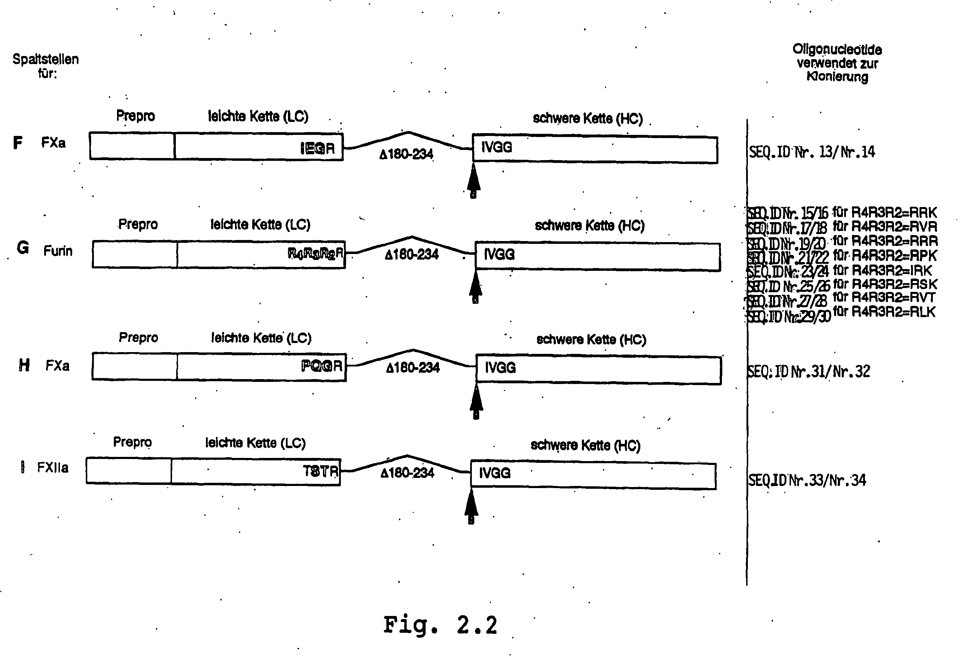

Bevorzugte Ausführungsformen der erfindungsemäßen Faktor X-Analoge sind dabei Faktor X-Analoge, die eine Modifikation aufweisen mit
a) R1=Ile, R2=Thr, R3=Leu, R4=Asn und gegebenenfalls R5=Asn und/oder R6=Asp und durch Faktor VIIa oder Faktor IXa prozessiert werden;
b) R1=Val, R2= Thr, R3=Phe, R4=Asp und gegebenenfalls R5=Asn und/oder R6=Phe und/oder R1=Ile oder Val (Fig. 2 A) und durch Faktor XIa prozessiert wird;
c) R1=Ile oder Val, R2=Phe, R3=Lys, R4=Ile und gegebenenfalls R5=Lys und/oder R6=Thr (Fig. 2 C) oder
   R1=Ile, R2=Thr, R3=Ser, R4=Thr und gegebenenfalls R5=Lys und/oder R6=Thr (Fig. 2 I) und durch Faktor XIIa prozessiert werden;
d) R1= Ile oder Val, R2=Thr, R3=Met, R4=Ser und gegebenenfalls R5=Ser und/oder R6=Leu (Fig. 2 D) und durch Kallikrein prozessiert wird;
e) R1=Ile, R2=Gly, R3=Gln, R4=Pro und gegebenenfalls R5=Lys und/oder R6=Ser (Fig. 2 H) oder
   R1=Ile, R2=Gly, R3=Glu, R4=Ile (Fig. 2 F) oder
   R1= Ile, R2=Thr, R3=Lys, R4= Met (Fig. 2 E) und durch Faktor Xa prozessiert werden;
f) R1=Ile, R2=Lys, R3=Arg, R4=Arg und gegebenenfalls R5=Glu und/oder R6=Leu oder
   R1=Ile, R2=Thr, R3=Val, R4=Arg und gegebenenfalls R5=Ala und/oder R6=Leu oder
   R1=Ile, R2=Arg, R3=Val, R4=Arg und gegebenenfalls R5=Gln und/oder R6=Leu oder
   R1=Ile, R2=Arg, R3=Arg, R4=Arg und gegebenenfalls R5=His und/oder R6=Leu oder
   R1=Ile, R2=Lys, R3=Pro, R4=Arg und gegebenenfalls R5=Asn und/oder R6=Leu oder
   R1=Ile, R2=Lys, R3=Arg, R4=Ile und gegebenenfalls R5=Arg und/oder R6=Leu oder
   R1=Ile, R2=Lys, R3=Ser und R4=Arg oder
   R1=Ile, R2=Thr, R3=Val und R4=Arg oder
   R1=Ile, R2=Lys, R3=Leu und R4=Arg (siehe alle Fig. 2 G),
wobei die unter f) genannten Sequenzen durch eine bibasische Endoprotease, wie etwa Kexin/Kex2, Furin/PACE, PC1/PC3, PC2, PC4, PACE 4, LPC/PC7 oder einem Derivat dieser Proteasen prozessiert werden.

Eine mögliche Auswahl von Modifikationen und Aminosäureaustauschern, die zu einer veränderten Proteasespezifität führen sind in Fig. 2 gezeigt.

Die Modifikationen können dabei beispielsweise durch gerichtete in vitro-Mutagenese oder PCR oder andere aus dem Stand der Technik bekannte gentechnische Methoden durchgeführt werden, die dazu geeignet sind, spezifisch eine DNA-Sequenz zu verändern, um gezielt Aminosäurenaustausche auszuführen.

Die Aktivierung des erfindungsgemäßen Faktor XΔ-Analogon zu einem Faktor Xa-Analogon erfolgt gemäß der vorliegenden Erfindung daher vorzugsweise durch eine Protease, ausgewählt aus der Gruppe der Endoproteasen, wie etwa Kexin/Kex2, Furin/PACE, PC1/PC3, PC2, PC4, PACE 4, LPC/PC7, der Serinproteasen, wie etwa Faktor IIa, Faktor VIIa, Faktor IXa, Faktor XIIa, Faktor XIa, Faktor Xa oder von Kallikrein, oder einem Derivat dieser Proteasen.

Die erfindungsgemäßen Faktor XΔ-Analoge liegen als einzelkettige Polypeptide in einer enzymatisch inaktiven Form vor. Erst durch Spaltung durch eine Protease in die zweikettige Form wird aktives Faktor Xa-Analogon erhalten. Die Modifikation erlaubt daher eine Aktivierung des inaktiven, einzelkettigen Faktor XΔ-Analogon-Polypeptids in die zweikettige, aktive Form.

Eine der Schwierigkeiten bei der Herstellung von aktivem Faktor Xa ist seine Instabilität, da durch Autokatalyse neben Faktor Xaα und Faktor Xaβ auch andere, inaktive Intermediate entstehen.

Zur Herstellung von im wesentlichen intakten, aktiven Faktor X/Xa- bzw. Faktor X/Xa-ähnlichen Molekülen wäre es daher wünschenswert, nur solche Proteine zu erhalten, die zu stabilen Endprodukten führen.

Es ist bekannt, daß eine bevorzugte Spaltstelle für die Prozessierung von Faktor Xaα (FXaα) zu Faktor Xaβ (FXaβ) zwischen Arg469/Gly470 liegt. Aufgrund von Untersuchungen von Eby et al., (1992, Blood. Vol. 80, Suppl. 1, 1214) wird neben einem prominenten carboxyterminalen Peptid (Aminosäurereste 476-487) von Faktor X ein weiteres kürzeres Peptid (Aminosäurereste 474 bis 477) gefunden, das durch Autokatalyse von Faktor Xaα entsteht. Um eine gezielte Prozessierung von intaktem Faktor X zu im wesentlichen aktivem Faktor Xa zu focussieren, ohne dabei inaktive Prozessierungs-Intermediate zu erhalten, weisen die erfindungsgemäßen Faktor XΔ-Analoge gegebenenfalls weitere Modifikationen auf.

Die erfindungsgemäßen Faktor XΔ-Analogon weisen daher gemäß einer besonderen Ausführungsform eine weitere Modifikation im C-terminalen Bereich der Faktor X-Aminosäuresequenz auf.

Gemäß einer Ausführungsform weist ein Faktor XΔ-Analogon der oben beschriebenen Art ein intaktes β-Peptid (FXΔa) auf. Die erfindungsgemäßen Faktor XΔ-Analogon besitzen dabei insbesondere eine Modifikation im Bereich der C-terminalen β-Peptidspaltstelle, die verhindert, daß nach Aktivierung von Faktor XΔ zu Faktor Xa-Analogon eine Abspaltung des β-Peptids von Faktor X erfolgt. Dadurch wird ein Faktor Xa-Molekül erhalten, das bis zu 100% als intaktes Faktor Xaα-Molekül isoliert werden kann.

Die Modifikation kann eine Mutation, Deletion oder Insertion im Bereich der Faktor X-Aminosäuresequenz zwischen Aminosäureposition Arg469 und Ser476 und gegebenenfalls von Lys370 sein. Bevorzugt ist jedoch eine Aminosäuresubstitution, durch die vermieden wird, daß durch den Aminosäureaustausch eine die Struktur und damit gegebenenfalls die Funktion und Aktivität des Proteins beeinflussende Faltung des Polypeptides erfolgt.

Gemäß einer Ausführungsform weisen die erfindungsgemäßen Faktor XΔ-Analogen einen Austausch einer der Aminosäuren an Position Arg469 und/oder Gly470, wobei Arg 469 vorzugsweise gegen Lys, His oder Ile und Gly470 vorzugsweise gegen Ser, Ala, Val oder Thr ausgetauscht ist.

Die erfindungsgemäßen Faktor XΔ-Analogen können neben einer Mutation an Position Arg469 und/oder Gly470 eine weitere Mutation an Position Lys370 und/oder Lys475 und/oder Ser476 aufweisen. Durch eine Aminosäuresubstitution an dieser(n) Position(en) wird eine Prozessierung von Faktor Xaα-Analogon zu Faktor Xaβ-Analogon bzw. C-terminal trunkierten Faktor Xa-Analogen vermieden, da die natürlicherweise vorkommende(n) Prozessierungssequenz(en) derart modifiziert ist (sind), daß eine gegebenenfalls autokatalytische Abspaltung eines Carboxy-terminalen Peptids nicht mehr erfolgen kann.

Gemäß einer anderen Ausführungsform weisen die erfindungsgemäßen Faktor X-Analogon eine Deletion des carboxyterminalen β-Peptids (FXΔβ) auf. Ein solches Faktor X-Analogon kann hergestellt werden, indem eine cDNA kodierend für Faktor XΔ-Analogon in einem rekombinanten Expressionssystem exprimiert wird, wobei nur die Sequenzen kloniert werden, die für die Aminosäuren Met1 bis Arg179/Ile235 bis Arg469 kodieren.

Gemäß einer weiteren Ausführungsform weisen die erfindungsgemäßen Faktor XΔ-Analoge ein Translationsstopsignal im C-terminalen Bereich der Faktor X-Sequenz auf. Das Translationsstopsignal ist dabei vorzugsweise an einer Position, die einer nach natürlicher Prozessierung entstehenden C-terminalen Aminosäure folgt. Das Translationsstopsignal ist daher vorzugsweise an Position der Aminosäure 470 der Faktor X-Sequenz, damit das endständige Arg469 des Faktor XΔβ erhalten bleibt. Dazu wird das für die Aminosäure Gly470 kodierende Kodon GGC gegen TAA, TAG oder TGA substituiert.

Ein weiterer Aspekt der vorliegenden Erfindung betrifft Faktor XΔ-Analoge, die durch Behandlung mit einer entsprechenden Protease in vitro in Faktor Xa-Analogon aktiviert werden, also die aktivierten Faktor XΔ-Analoge. Abhängig vom Faktor XΔ-Analogon, das eingesetzt und aktiviert wird, wird ein Faktor XaΔ-Analogon erhalten, das am C-terminalen Ende der leichten Kette entsprechende Aminosäure-Modifikationen gegenüber der natürlichen Faktor Xa-Sequenz aufweist. Die Modifikationen sind erfindungsgemäß jedoch so ausgewählt, daß sie die biologische Aktivität nicht beeinträchtigen.

Weist ein solches Faktor X-Analogon gegebenenfalls zusätzlich ein Translationsstopsignal im C-terminalen Bereich des β-Peptids auf, so werden modifizierte Faktor Xaβ-Moleküle gewonnen. Wird jedoch ein Faktor X-Analogon eingesetzt, das Modifikation(en) innerhalb der β-Peptidsequenz aufweist, die dazu führt(en), daß das β-Peptid nicht abgespalten wird, so wird ein Faktor Xaα-Analogon mit einem Aminosäureaustausch im C-Terminus des Moleküls erhalten.

Die erfindungsgemäßen Faktor XΔ-Analoge weisen ausschließlich Modifikationen auf, die die Spezifität für die Aktivierbarkeit ändern und nicht signifikant die Aktivität beeinflussen. Es werden daher in jedem Fall biologisch und funktionell aktive Faktor Xa-Moleküle bzw. Faktor Xa-Analoge gewonnen.

Die Aktivierung in vitro kann durch eine Protease ausgewählt aus der Gruppe der Endoproteasen, wie etwa Kexin/Kex2, Furin/PACE, PC1/PC3, PC2, PC4, PACE 4, LPC/PC7, der Serinproteasen, wie etwa Faktor IIa, Faktor VIIa, Faktor IXa, Faktor XIIa, Faktor XIa, Faktor Xa oder von Kallikrein, oder einem Derivat dieser Proteasen, erfolgen. Es liegt jedoch im Rahmen der vorliegenden Erfindung, jede beliebige Protease, außer RVV oder Trypsin, einzusetzen, sofern sie geeignet ist, das erfindungsgemäße Faktor XΔ-Analogon zu Faktor Xa-Analogon zu prozessieren.

Obwohl beispielsweise von Wolf et al. (1991, J. Biol. Chem. 266:13726-137309) vermutet wurde, daß eine Endopeptidase wie Kex2, Furin oder PACE an der Prozessierung der von dieser Gruppe beschriebenen Faktor Xa-Deletionsmutante beteiligt ist, geben sie keinerlei Hinweise über den Einfluß einer dieser Proteasen bei der Prozessierung von Faktor X. Ebenso wird in der US 5,660,950 die rekombinante Herstellung von PACE und die Verwendung der Protease zur Verbesserung der Prozessierung von Vitamin-K abhängigen Proteinen beschrieben. In einer Reihe von Aufzählungen mit anderen Blutfaktoren wird auch Faktor X genannt, wobei jedoch diese Aussage verifizierende Daten fehlen.

Im Rahmen der vorliegenden Erfindung wurde zum erstenmal eindeutig gezeigt, daß eine der für den Reifungsprozeß von Faktor X notwendige Protease eine bibasische Endoprotease, insbesondere endogen vorkommendes Furin, ist. In vivo vermittelt die Endoprotease in erster Linie die Spaltung des einzelkettigen Faktor X-Moleküls in die reife Form bestehend aus schwerer und leichter Kette. In vitro vermittelt sie zusätzlich die Abspaltung der Faktor X Pro-Peptid-Sequenz (Beispiel 2).

Gemäß einer besonderen Ausführungsform wird ein Faktor XΔ-Analogon bereitgestellt, das vorzugsweise in gereinigter Form als einzelkettiges Molekül vorliegt. Faktor XΔ-Analoge, die im modifizierten Bereich eine Schnittstelle für eine in rekombinanten Zellen nicht vorkommende Protease aufweisen, werden nach Expression als einzelkettiges Molekül erhalten. Das einzelkettige Faktor XΔ-Molekül zeichnet sich insbesondere durch seine hohe Stabilität und molekulare Integrität aus. Bisher konnte ein einzelkettiges, inaktives Faktor XΔ-Molekül nicht in gereinigter Form isoliert werden, da es in rekombinanten Zellen in Faktor Xa und eine Reihe von weiteren, auch inaktiven, Intermediaten prozessiert wird (Wolf et al., 1991, J. Biol. Chem. 266:13726-13730). Das isolierte einzelkettige Faktor XΔ-Analogon kann durch spezifische Prozessierung direkt in die zweikettige Faktor Xa-Analogon-Form aktiviert werden. Dies kann dadurch erfolgen, daß ein aus einer rekombinanten Zelle isoliertes einzelkettiges Faktor XΔ-Molekül mit einer die im Faktor XΔ-Analogon befindliche Aktivierungsstelle spaltenden Protease in Kontakt gebracht wird. Wird etwa ein Faktor XΔ-Analogon mit einer Furin-Aktivierungsstelle in einer Furin-defizienten Zelle exprimiert, so kann es als einzelkettiges Faktor XΔ-Analogon isoliert und durch In-Kontakt-bringen mit einer bibasischen Protease, wie Furin/PACE oder Kex2 in aktives, zweikettiges Faktor XΔa-Analogon prozessiert werden. Faktor XΔ-Analoge mit einer Prozessierungsstelle für eine Serinprotease oder Kallikrein können auch in Furinexprimierenden Zellen als einzelkettiges Molekül isoliert und anschließend mit der Serinprotease in aktives Faktor Xa-Analogon prozessiert werden.

Ein derart erhaltenes Faktor Xa-Analogon weist aufgrund der selektiven und gerichteten Prozessierungsreaktion eine hohe Stabilität und strukturelle Integrität auf und ist insbesondere frei von inaktiven Faktor X/Xa-Analogon-Intermediaten und autoproteolytischen Abbauprodukten.

Das erfindungsgemäße Faktor XΔ-Analogon wird gemäß der vorliegenden Erfindung sowohl in Form eines Faktor XΔa mit intaktem β-Peptid als auch in Form eines Faktor XΔ-Analogon mit einer Deletion des β-Peptids zur Verfügung gestellt.

Ein weiterer Aspekt der vorliegenden Erfindung betrifft die rekombinante DNA kodierend für die erfindungsgemäßen Faktor XΔ-Analoge. Die rekombinante DNA resultiert nach Expression in einem Faktor XΔ-Analogon mit einer Aminosäuresequenz entsprechend dem humanen Faktor X, außer einer Deletion der Aminosäuren von Arg180 bis Arg234 und einer Modifikation, die eine Prozessierung und Aktivierung in aktiven Faktor Xa-Analogon, sowohl mit intaktem als auch deletiertem β-Peptid, ermöglicht.

Ein weiterer Aspekt der Erfindung betrifft eine Präparation enthaltend ein gereinigtes Faktor XΔ-Analogon, das eine Deletion der Aminosäuren von Arg180 bis Arg234 und einer Modifikation der Aminosäuren im Bereich zwischen Gly173 und Arg179 und gegebenenfalls von Ile235 aufweist. Die Modifikation führt dabei zu einer neuen, nicht-natürlicherweiser an dieser Position im Polypeptid vorkommenden Erkennungs- bzw. Spaltstelle für eine Protease, die das Polypeptid normalerweise nicht an dieser Stelle prozessiert. Die Präparation kann dabei eine gereinigte Präparation enthaltend einzelkettiges Faktor XΔ-Analogon sein, wobei die Polypeptide aus einem Zellkultursystem entweder nach Isolierung aus dem Zellkulturüberstand oder aus einem Extrakt einer Zellkultur erhalten werden. Ein aus einem Zellkultursystem vorgereinigtes rekombinantes Faktor XΔ-Analogon kann über aus dem Stand der Technik bekannte Verfahren weiter gereinigt werden. Dazu eignen sich insbesondere chromatographische Verfahren, wie Gelfiltration, Ionenaustauscher- oder Affinitätschromatographie.

Gemäß einer Ausführungsform enthält die erfindungsgemäße Präparation das Faktor XΔ-Analogon als einzelkettiges Molekül in enzymatisch inaktiver Form, wobei das Faktor XΔ-Analogon mit einer Reinheit von mindestens 80%, vorzugsweise mindestens 90%, besonders bevorzugt von mindestens 95% vorliegt, und in der gereinigten Präparation keine inaktiven, proteolytischen Intermediate von Faktor X/Xa-Analogon enthalten sind.

Gemäß einem besonderen Aspekt enthält die Präparation einzelkettiges Faktor XΔ-Analogon mit einer Modifikation, die eine Aktivierung zu Faktor Xa-Analogen durch eine der Protease, ausgewählt aus der Gruppe der bibasischen Endoproteasen, wie etwa Kexin/Kex2, Furin/PACE, PC1/PC3, PC2, PC4, PACE 4, LPG/PC7, der Serinproteasen, wie etwa Faktor IIa, Faktor VIIa, Faktor IXa, Faktor XIIa, Faktor XIa, Faktor Xa oder von Kallikrein, oder einem Derivat dieser Proteasen erlaubt. Die Aktivierung erfolgt dabei durch In-Kontakt-bringen des Faktor XΔ-Analogons mit der entsprechenden Protease, die bei der modifizierten Sequenz spaltet, wodurch ein Faktor Xa-Analoge erhalten wird.

In der erfindungsgemäßen Präparation kann das Faktor XΔ-Analogon entweder als Faktor XΔα (FXΔα) mit intaktem β-Peptid oder mit einer Deletion des β-Peptids als Faktor XΔβ oder anderer C-terminalen Deletionen vorliegen.

Gemäß einer weiteren Ausführungsform enthält die erfindungsgemäße Präparation das Faktor XΔ-Analogon vorzugsweise als einzelkettiges Molekül in isolierter Form. Dazu wird beispielsweise durch rekombinante Herstellung Faktor XΔ-Analogon als einzelkettiges Molekül mit einer Modifikation, die eine Aktivierung zu Faktor Xa-Analogon in vitro erlaubt, gewonnen. Die Aktivierung von Faktor XΔ-Analogon zu Faktor Xa-Analogon kann dabei erfolgen durch In-Kontakt-bringen des Faktor X-Analogons mit einer Protease, ausgewählt aus der Gruppe der bibasischen Endoproteasen, wie etwa Kexin/Kex2, Furin/PACE, PC1/PC3, PC2, PC4, PACE 4, LPC/PC7, der Serinproteasen, wie etwa Faktor IIa, Faktor VIIa, Faktor IXa, Faktor XIIa, Faktor XIa, Faktor Xa oder von Kallikrein, oder einem Derivat dieser Proteasen. Die Protease kann dabei an einen Träger immobilisiert sein.

Die erfindungsgemäße Präparation kann als Ausgangsmaterial zur Herstellung und Gewinnung von Faktor Xa-Analogen dienen. Dazu wird in einem großtechnischen Ansatz die Präparation, enthaltend einzelkettiges Faktor XΔ-Analogon mit einer gegebenenfalls immobilisierten Protease unter Bedingungen, die eine optimale Aktivierung von Faktor XΔ-Analogon zu Faktor Xa-Analogon erlauben, in Kontakt gebracht und Faktor Xa-Analogon erhalten. Das so gewonnene Faktor Xa-Analogon kann anschließend über allgemein bekannte Methoden gereinigt und zu einer pharmazeutischen Zusammensetzung mit Faktor Xa-Aktivität formuliert werden.

Gemäß einem weiteren Aspekt der vorliegenden Erfindung wird eine Präparation, enthaltend ein Faktor Xa-Analogon mit hoher Stabilität und struktureller Integrität, das insbesondere frei ist von inaktiven Faktor X/Xa-Analogon-Intermediaten und autoproteolytischen Abbauprodukten, bereitgestellt und das dadurch erhältlich ist, daß ein Faktor XΔ-Analogon der oben beschriebenen Art aktiviert und zu einer entsprechenden Präparation zubereitet wird.

Gemäß einer besonderen Ausführung enthält die Präparation, enthaltend das gereinigte, einzelkettige oder zweikettige Faktor XΔ-Analogon, einen physiologisch akzeptablen Träger und ist gegebenenfalls als pharmazeutisches Präparat formuliert. Die Formulierung kann in an sich üblicher Weise erfolgen und mit einem Puffer, enthaltend Salze, wie NaCl, CaCl₂, und Aminosäuren, wie Glycin und/oder Lysin, bei einem pH im Bereich von 6 bis 8 gemischt und als pharmazeutisches Präparat formuliert sein. Die gereinigte Präparation, enthaltend Faktor X-Analogon kann als fertige Lösung, Lyophilisat oder tiefgefroren bis zum Endgebrauch als lagerfähiges Produkt bereitgestellt werden. Vorzugsweise erfolgt die Lagerung der Präparation in lyophiliserter Form und wird mit einer entsprechenden Rekonstitutionslösung in eine optisch klare Lösung gelöst.

Die Präparation gemäß der vorliegenden Erfindung kann jedoch auch als Flüssigpräparat bzw. in flüssig-tiefgefrorener Form zur Verfügung gestellt werden.

Die erfindungsgemäße Präparation ist besonderes stabil, d.h. sie kann auch in gelöster Form über längere Zeit vor der Applikation stehen gelassen werden. Es hat sich gezeigt, daß die erfindungsgemäße Präparation für mehrere Stunden bis Tage keinerlei Aktivitätsverlust zeigt.

Die erfindungsgemäße Präparation kann in einer geeigneten Vorrichtung, vorzugsweise einer Applikationsvorrichtung, in Kombination mit einer Protease, ausgewählt aus der Gruppe der Endoproteasen, wie etwa Kexin/Kex2, Furin/PACE, PC1/PC3, PC2, PC4, PACE 4, LPC/PC7, der Serinproteasen, wie etwa Faktor IIa, Faktor VIIa, Faktor IXa, Faktor XIIa, Faktor XIa, Faktor Xa oder von Kallikrein, oder einem Derivat dieser Proteasen, vorliegen.

Die erfindungsgemäße Präparation, enthaltend ein Faktor XΔ-Analogon in Kombination mit einer Protease, die in der Lage ist, das Faktor XΔ-Analogon zu Faktor Xa-Analogon zu aktivieren, kann als Kombinationspräparat bereitgestellt werden, bestehend aus einem Behälter enthaltend eine an einen Träger immobilisierte Protease, gegebenenfalls in Form einer Mini-Säule oder einer mit einer immobilisierten Protease bestückten Spritze und einem Behälter enthaltend die pharmazeutische Präparation mit Faktor XΔ-Analogon. Zur Aktivierung des Faktor XΔ-Analogons wird die Faktor XΔ-Analogon-haltige Lösung beispielsweise über die immobilisierte Protease gedrückt. Die Faktor XΔ-Analogon haltige Lösung ist dabei während der Lagerung des Präparates vorzugsweise von der immobilisierten Protease räumlich getrennt. Die erfindungsgemäße Präparation kann im gleichen Behälter wie die Protease sein, wobei die Komponenten jedoch durch eine impermeable Trennwand, die bei etwaigem Gebrauch leicht zu entfernen ist, räumlich getrennt sind. Die Lösungen können auch in eigenen Behältern aufbewahrt werden und erst kurz vor Anwendung miteinander in Kontakt gebracht werden.

In einer besonderen Ausführungsform ist die zur Aktivierung eingesetzte Protease eine natürlicherweise bei der Blutgerinnung beteiligte Serinprotease, wie etwa Faktor XIIa, die dann vor der Applikation nicht vom aktivierten Faktor Xa-Analogon abgetrennt werden muß, sondern mit ihm appliziert werden kann.

Die Aktivierung von Faktor XΔ-Analogon zu Faktor Xa-Analogon kann kurz vor dem direkten Gebrauch, also vor der Applikation am Patienten erfolgen. Die Aktivierung kann durch In-Kontakt-bringen mit einer immobilisierten Protease oder durch Mischen von Lösungen, enthaltend einerseits eine Protease und andererseits Faktor XΔ-Analogon, erfolgen. Es ist daher möglich, die beiden Komponenten getrennt voneinander in Lösung zu halten und durch eine geeignete Vorrichtung, bei der die Komponenten während des Durchlaufs in Kontakt kommen, zu mischen, wodurch Faktor XΔ-Analogon zu Faktor Xa-Analogon aktiviert wird. Dem Patienten wird so ein Gemisch von Faktor Xa und einer weiteren Serinprotease, die die Aktivierung bewirkt hat, verabreicht werden. Hierbei ist insbesondere auf die Dosierung zu achten, da durch die zusätzliche Gabe einer Serinprotease auch endogener Faktor X aktiviert wird und damit die Gerinnungszeit verkürzt sein kann.

Gemäß einer bevorzugten Ausführungsform wird die pharmazeutische Präparation in einer geeigneten Vorrichtung, vorzugsweise einer Applikationsvorrichtung, entweder in flüssig-gefrorener oder lyophilisierter Form zur Verfügung gestellt. Eine geeignete Applikationsvorrichtung kann dabei ein gemäß der AT 366 916 oder der AT 382 783 beschriebener Doppelkammerspritzenkörper sein.

Gemäß einem weiteren Aspekt der Erfindung enthält die erfindungsgemäße Präparation gegebenenfalls als weiteren Bestandteil einen Blutfaktor in Form des Zymogens oder einer aktiven Serinprotease. Bevorzugt sind dabei als weitere Bestandteile solche Komponenten mit FEIB-Aktivität. Dazu gehören insbesondere Faktor II, Faktor VII, Faktor IX, Faktor VIII, Faktor V und/oder die aktiven Serinproteasen davon. Weitere Bestandteile können jedoch auch Phospholipide, Ca-Ionen u.a. sein. Gemäß einer besonderen Ausführungsform der Erfindung enthält die erfindungsgemäße Präparation mindestens eine weitere Komponente mit FEIB-Aktivität.

Die erfindungsgemäße Präparation kann als pharmazeutische Präparation mit Faktor Xa-Aktivität als Einkomponenten-Präparat oder in Kombination mit anderen Faktoren als Mehrkomponentenpräparat zur Verfügung gestellt werden.

Vor der Aufbereitung in eine pharmazeutische Präparation wird das gereinigte Protein den üblichen Qualitätskontrollen unterzogen und in eine therapeutisch verabreichbare Form gebracht. Insbesondere wird bei der rekombinanten Herstellung das gereinigte Präparat auf Abwesenheit von zellulären und vom Expressionsvektor stammenden Nukleinsäuren getestet, vorzugsweise gemäß einem Verfahren, wie es in der EP 0 714 987 beschrieben ist.

Da prinzipiell jedes biologische Material mit infektiösen Keimen kontaminiert sein kann, wird zur Herstellung eines sicheren Präparates die Präparation gegebenenfalls zur Inaktivierung bzw. Abreicherung von Viren behandelt.

Ein weiterer Aspekt der Erfindung betrifft die Verwendung einer Präparation der oben beschriebenen Art zur Herstellung eines Arzneimittels. Ein Arzneimittel, enthaltend ein erfindungsgemäßes Faktor XΔ-Analogon und entsprechend aktiviertes Faktor X-Analogon, eignet sich insbesondere zur Behandlung von Patienten mit Störungen der Blutgerinnung, wie etwa Hämophilie-Patienten oder Patienten, welche inhibierende Antikörper gegen das verabreichte Therapeutikum entwickelt haben, z.B. gegen Faktor VIII oder Faktor IX.

Ein weiterer Aspekt der Erfindung betrifft ein Verfahren zur Herstellung des Faktor XΔ-Analogon und eine Präparation, enthaltend das erfindungsgemäße Faktor XΔ-Analogon. Dazu wird die für das Faktor XΔ-Analogon kodierende Sequenz in ein geeignetes Expressionssystem eingebracht und entsprechende Zellen mit der rekombinanten DNA transfiziert. Vorzugsweise werden permanente Zellinien etabliert, die Faktor XΔ-Analogon exprimieren. Die Zellen werden unter optimalen Bedingungen für die Genexpression kultiviert und Faktor X-Analoge entweder aus einem Extrakt einer Zellkultur oder dem Zellkulturüberstand isoliert. Das rekombinante Molekül kann durch alle bekannten chromatographischen Verfahren, wie Anionen- oder Kationenaustauscher-, Affinitäts- oder Immunaffinitätschromatographie, oder einer Kombination davon, weiter gereinigt werden.

Zur Herstellung der erfindungsgemäßen Faktor XΔ-Analoge wird die komplette für Faktor X kodierende cDNA in einen Expressionsvektor kloniert. Dies erfolgt entsprechend allgemein bekannten Klonierungstechniken. Die für Faktor X kodierende Nukleotidsequenz wird anschließend derart modifiziert, daß die für die Aminosäuren Arg180 bis Arg234 kodierenden Sequenzen deletiert und Aminosäuren im Bereich zwischen Gly173 und Arg179, gebenenfalls Ile235 derart verändert werden, daß ein Faktor XΔ-Molekül der oben beschriebenen Art hergestellt werden kann. Dies erfolgt durch aus dem Stand der Technik bekannte gentechnische Methoden, wie gerichtete in vitro-Mutagenese, Deletion von Sequenzen, beispielsweise durch Restriktionsverdau durch Endonukleasen und Insertion anderer veränderter Sequenzen, oder durch PCR. Die so hergestellten Faktor XΔ-Mutanten werden dann in ein für die rekombinante Expression geeignetes Expressionssystem inseriert und exprimiert.

Die erfindungsgemäßen Faktor XΔ-Analoge können ebenfalls durch chemische Synthese hergestellt werden.

Die Faktor XΔ-Analoge werden vorzugsweise durch rekombinante Expression hergestellt. Die gentechnische Herstellung kann mit allen gängigen Expressionssystemen, wie z.B. permanenten Zelllinien oder viralen Expressionssystemen, erfolgen. Die permanenten Zellinien werden hergestellt durch stabile Integration der Fremd-DNA in das Wirtszellchromosom von z.B. Vero, MRC5, CHO, BHK, 293, Sk-Hep1, insbesondere Leber- und Nierenzellen, oder durch einen episomalen Vektor, abgeleitet von z.B. Papilloma Virus. Virale Expressionssysteme, wie beispielsweise Vaccinia Virus, Baculovirus oder retrovirale Systeme können ebenfalls eingesetzt werden. Als Zellinien werden allgemein Vero, MRC5, CHO, BHK, 293, Sk-Hepl, Drüsen-, Leber- und Nierenzellen eingesetzt. Als eukaryotische Expressionssysteme können auch Hefen, endogene Drüsen (z.B. Drüsen transgener Tiere) und andere Zelltypen verwendet werden. Natürlich können auch transgene nicht menschliche Tiere zur Expression der erfindungsgemäßen Polypeptide oder Derivaten davon verwendet werden. Zur Expression der rekombinanten Proteine haben sich im speziellen CHO-DHFR⁻-Zellen bewährt (Urlaub et al., Proc. Natl. Acad. Sci., USA, 77:4216-4220, 1980).

Zur rekombinanten Herstellung der erfindungsgemäßen Faktor XΔ-Analoge können auch prokaryontische Expressionssysteme eingesetzt werden. Hierzu eignen sich insbesondere Systeme, die eine Expression in E. coli oder B. subtilis erlauben.

Die Faktor XΔ-Analogen werden in den entsprechenden Expressionssystemen unter der Kontrolle eines geeigneten Promotors exprimiert. Im Fall der Expression in Eukaryonten eignen sich dazu alle bekannten Promotoren, wie SV40-, CMV-, RSV-, HSV-, EBV-, β-Actin-, hGH oder induzierbare Promotoren wie z.B. hsp- oder Metallothionein-Promotor. Vorzugsweise werden die Faktor X-Analoge unter Kontrolle des β-Actin-Promotors in CHO-DHFR⁻-Zellen exprimiert.

Gemäß einer Ausführungsform der Erfindung umfaßt das Verfahren zur Herstellung der erfindungsgemäßen Präparation die Schritte: Bereitstellen einer für ein Faktor XΔ-Analogon kodierenden DNA, Transformation einer Zelle mit der rekombinanten DNA, Expression des Faktor X-Analogons, gegebenenfalls in Gegenwart einer Protease, Isolieren des Faktor X-Analogons und gegebenenfalls Reinigung über ein chromatographisches Verfahren.

Gemäß einer Ausführungsform des Verfahrens wird das Faktor Xa-Analogon direkt als zweikettiges Molekül isoliert. Dazu wird ein Faktor XΔ-Analogon, das eine Modifikation aufweist, die eine Prozessierung durch eine bibasische Protease wie Furin erlaubt, in einer Zelle exprimiert und das Faktor XΔ-Analogon in zweikettiges Faktor Xa-Analogon prozessiert. Die Zelle ist dabei vorzugsweise eine Zelle, die eine für die Prozessierung fähige Protease, etwa eine bibasische Protease, wie Furin oder ein Derivat davon, exprimiert. Gegebenenfalls kann zur Erhöhung bzw. Verbesserung der Prozessierungseffizienz die Zelle derart modifiziert werden, daß sie die Protease verstärkt exprimiert. Dies kann beispielsweise durch Co-Expression einer entsprechenden bibasischen Endoprotease, etwa Furin/PACE, Kex2 oder einem Derivat davon erfolgen. Das erfindungsgemäße Faktor XΔ-Analogon kann ebenfalls in einer Zelle exprimiert werden, die eine normale oder damit für die Prozessierung suboptimale endogene Konzentration einer Protease aufweist und demzufolge eine unvollständige Prozessierung in die zweikettige aktive Form stattfindet. Die anschließende Prozessierung zu Faktor Xa-Analogon erfolgt in diesem Fall, sofern einkettiges Faktor X-Analogon in den Zellkulturüberstand sezerniert wird, wie oben beschrieben, durch Co-Kultivierung mit Protease-exprimierenden Zellen oder In-Kontakt-bringen mit einer, gegebenenfalls immobilisierten, Protease. Der Zellüberstand kann auch über eine Trägermatrix, an die eine Protease gebunden ist, gepumpt werden, wodurch im Eluat zweikettiges Faktor Xa-Analogon erhalten wird.

Das so erhaltene Faktor Xa-Analogon kann anschließend isoliert, gereinigt und bis zur weiteren Verwendung, wie oben beschrieben, gegebenenfalls als pharmazeutische Zusammensetzung formuliert und stabil gelagert werden. Die Reaktionsbedingungen für die Prozessierungsreaktion und die Aktivierung können ohne weiteres vom Fachmann je nach Versuchsanordnung der gegebenen Rahmenbedingungen optimiert werden. Dabei ist für die Kontaktdauer die Fließgeschwindigkeit der vorliegenden Reaktanden von besonderer Bedeutung. Diese sollte zwischen 0,01 ml/min und 1 ml/min liegen. Als weitere Parameter sind Temperatur, pH-Wert und Elutionsbedingungen von Bedeutung. Nach dem Durchlauf kann Faktor Xa-Analogon gegebenenfalls über selektive Chromatographie weiter gereinigt werden. Die Durchführung des Verfahrens mit jeweils an einen Träger gebundener Protease ist deshalb von besonderem Vorteil, da die Reaktionsanordnung durch Verwendung eines Trägers, vorzugsweise von Chromatographiesäulen, einen zusätzlichen Reinigungsschritt ermöglicht.

Gemäß einer Ausführungsform erfolgt die Aktivierung durch einen chromatographischen Schritt, bei dem die Protease an einen Träger immobilisiert ist. Gereinigtes einzelkettiges Faktor XΔ-Analogon wird dazu über eine Matrix, an die die Protease gebunden ist, geleitet und aus dem Eluat gereinigtes Faktor Xa-Analogon isoliert.

Gemäß einem Aspekt der Erfindung wird eine Präparation, enthaltend aktives Faktor Xa-Analogon, dadurch erhalten, daß ein wie oben beschrieben hergestelltes Faktor XΔ-Analogon einem Prozessierungs-/Aktivierungsschritt unterzogen wird und das aktivierte Polypeptid zu einer gereinigten Präparation, die gegebenenfalls als pharmazeutische Zusammensetzung formuliert ist, weiterverarbeitet wird.

Gemäß einem weiteren Aspekt zur Herstellung eines Präparates, enthaltend einzelkettiges Faktor XΔ-Analogon, wird etwa das Faktor XΔ-Analogon, das eine Prozessierungssequenz für eine bibasische Protease aufweist, in einer Zelle exprimiert, die eine Endoprotease-Defizienz aufweist. Die Zelle weist dabei vorzugsweise eine Defizienz einer bibasischen Endoprotease, wie etwa Kexin, Furin, PACE oder homologe Derivate davon auf. Aus einer solchen Endoprotease-Defizienten-Mutantenzelle kann Faktor XΔ-Analogon als einzelkettiges Molekül isoliert weren. Faktor XΔ-Analoge, die eine Prozessierungsstelle für eine Serinprotease aufweisen, können in jeder herkömmlichen Zelle, auch Furin-positiven Zelle, exprimiert werden und als einzelkettiges Molekül isoliert werden.

Ein derart isoliertes und gegebenenfalls gereinigtes Faktor X-Analogon wird anschließend mit einer Protease ausgewählt aus der Gruppe der Endoprotease, wie etwa Kexin/Kex2, Furin/PACE, PC1/PC3, PC2, PC4, PACE 4, LPC/PC7, der Serinproteasen, wie etwa Faktor IIa, Faktor VIIa, Faktor IXa, Faktor XIIa, Faktor XIa, Faktor Xa oder von Kallikrein, oder einem Derivat dieser Proteasen, in Kontakt gebracht, unter Bedingungen unter denen einzelkettiges Faktor X-Analogon zu Faktor Xa-Analogon gespalten und aktiviert wird.

Mit den erfindungsgemäßen Faktor XΔ-Analogen, die durch einen, wie oben beschriebenen, Prozess zu Faktor Xa-Analogen aktiviert werden, wird gereinigtes Faktor Xa-Analogon mit hoher Stabilität und struktureller Integrität und insbesondere frei von inaktiven Faktor X/Xa-Intermediaten, gewonnen.

Die Erfindung wird anhand der nachfolgenden Beispiele und der Zeichnungsfiguren näher beschrieben, wobei sie jedoch nicht auf diese besonderen Ausführungsbeispiele beschränkt ist.

Beispiel 1 beschreibt die Konstruktion und Expression eines rFaktor X; Beispiel 2 beschreibt die Prozessierung von rFaktor X in schwere und leichte Kette durch Furin; Beispiel 3 beschreibt die Prozessierung von pro-Faktor X mittels immobilisierter Protease; Beispiel 4 beschreibt die Aktivität des in vitro prozessierten rFaktor X; Beispiel 5 beschreibt die Expression von rFaktor X in Furin-defizienten Zellen; Beispiel 6 beschreibt die Konstruktion und Expression von rFaktor XΔ-Analogen; Beispiel 7 beschreibt die Bestimmung der N-Termini der Faktor X-Prozessierungsprodukte; Beispiel 8 beschreibt die Expression und Charakterisierung des FX-Analogon mit der Stelle Arg-Val-Thr-Arg/Ile (rFXΔ^{RVTR/I}); Beispiel 9 beschreibt die in vitro-Aktivierung des rFXΔ^{RVTR/I}-Proteins durch rFurin-Derivate.

Es zeigen:
- Figur 1:: Nukleotid-und Aminosäuresequenz von Faktor X
- Figur 2:: Schematische Darstellung der Faktor XΔ-Analoge mit modifizierten Proteasen-Schnittstellen
- Figur 3:: Schematische Darstellung des Expressionsvektors phAct-rFX
- Figur 4:: Westernblot-Analyse von rFaktor X exprimiert in CHO-Zellen vor und nach Amplifikation
- Figur 5:: Westernblot-Analyse von rFaktor X nach in vitro-Spaltung mit Furinderivaten
- Figur 6:: Westernblot-Analyse von rFaktor X-Molekülen exprimiert in Furin-haltigen und Furin-defizienten Zellen
- Figur 7:: Schematische Darstellung der rFaktor XΔ-Analogon-Konstrukte mit veränderten C-Termini der schweren Kette
- Figur 8:: Schematische Darstellung der N-Termini der rFaktor X-Prozessierungsprodukte aus CHO-, CHO/rFurin und Furin-defizienten Zellen
- Figur 9:: Westernblot-Analyse von rFaktor XΔ^{RVTR/I}, exprimiert in CHO-Zellen
- Figur 10:: Westernblot-Analyse von rFaktor XΔ^{RVTR/I} nach in vitro-Aktivierung mit Furinderivat.

Die Expressionsvektoren wurden mittels Standard-Klonierungstechniken (Maniatis et al., "Molecular Cloning"- A Laboratory Manual, Cold Spring Harbor Laboratory, Cold Spring Harbor, New York, USA, 1983) hergestellt. Die Herstellung von DNA-Fragmenten mittels Polymerase-Ketten-Reaktion (PCR) erfolgte durch allgemeine Methoden (Clackson et al., 1991, PCR A practical approach. ED. McPherson, Quirke, Taylor, S. 187-214).

### Beispiel 1 :

### Expression und Prozessierung von einzelkettigem rFX zu rFX leichte/schwere Kette

### a. Herstellung des rFX-Expressionsvektors

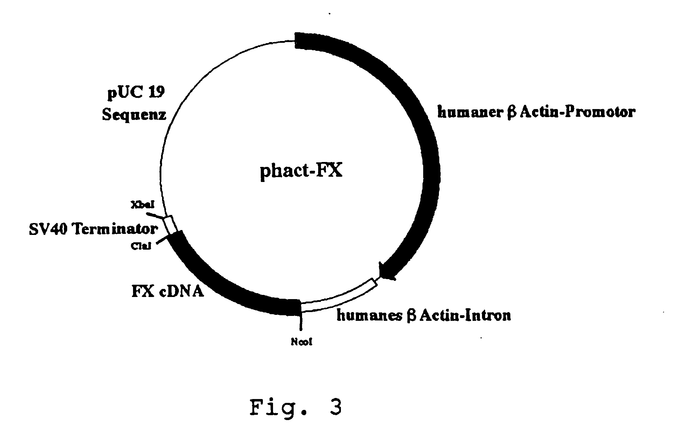

Zur Herstellung von rekombinantem FX (rFX) wurde die cDNA von FX aus einer humanen Leber Lambda-cDNA-Bank, wie von Messier et al. beschrieben (1991, Gene 99:291-294), isoliert. Aus einem positiven Klon wurde mittels PCR mit Oligonukleotid #2911 (5'-ATTACTCGAGAAGCTTACCATGGGGCGCCCACTG-3') (SEQ. ID. Nr. 1) als 5'-Primer und Oligonukleotid #2912 (5'-ATTACAATTGCTGCAGGGATCCAC-3') (SEQ. ID. Nr. 2) als 3'-Primer ein DNA-Fragment amplifiziert, das die 1,467kB FX-kodierende Sequenz sowie 39bp der 3'-nichttranslatierten Region, flankiert von einer XhoI-Schnittstelle, am 5'-Ende und einer MfeI-Schnittstelle am 3'-Ende enthält. Zusätzlich wurde durch den Primer #2911 die Sequenz ACC vor das ATG des FX eingebaut, so daß eine optimale Kozak-Translationsinitiations-Sequenz ensteht. Anschließend wurde dieses PCR-Produkt als XhoI/MfeI-Fragment in den mit SalI und EcoRI geschnittenen Expressionsvektor phAct kloniert. Das resultierende Expressionsplasmid wurde mit phAct-rFX bezeichnet (Figur 3). Der Expressionsvektor phAct umfaßt den humanen beta-Actin-Promoter, 78bp 5 ' UTR sowie das Intron, eine multiple Klonierungsschnittstelle und die SV40-Polyadenylierungsstelle.

### b. Expression von rFX in CHO-Zellen

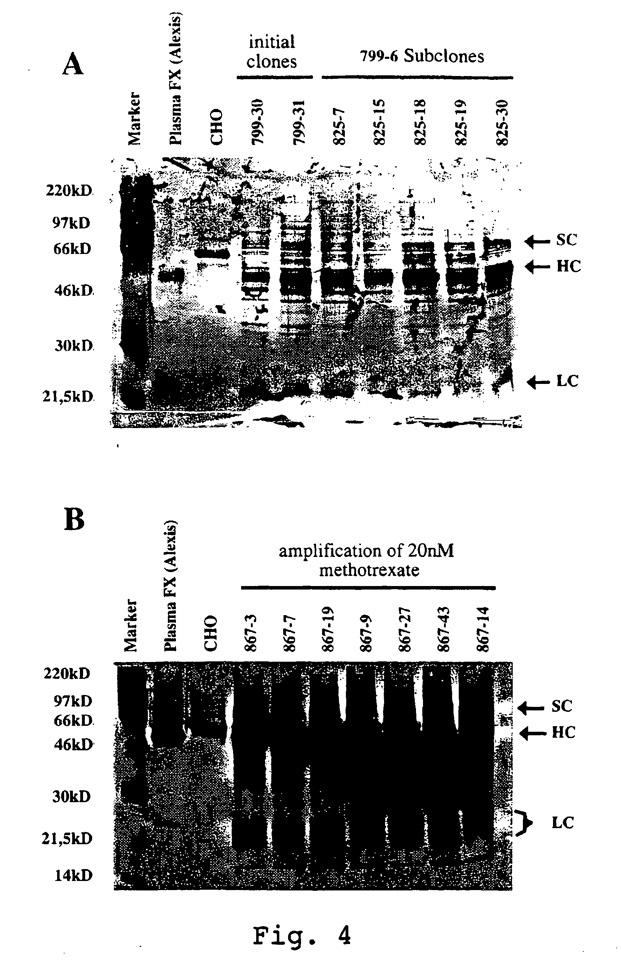

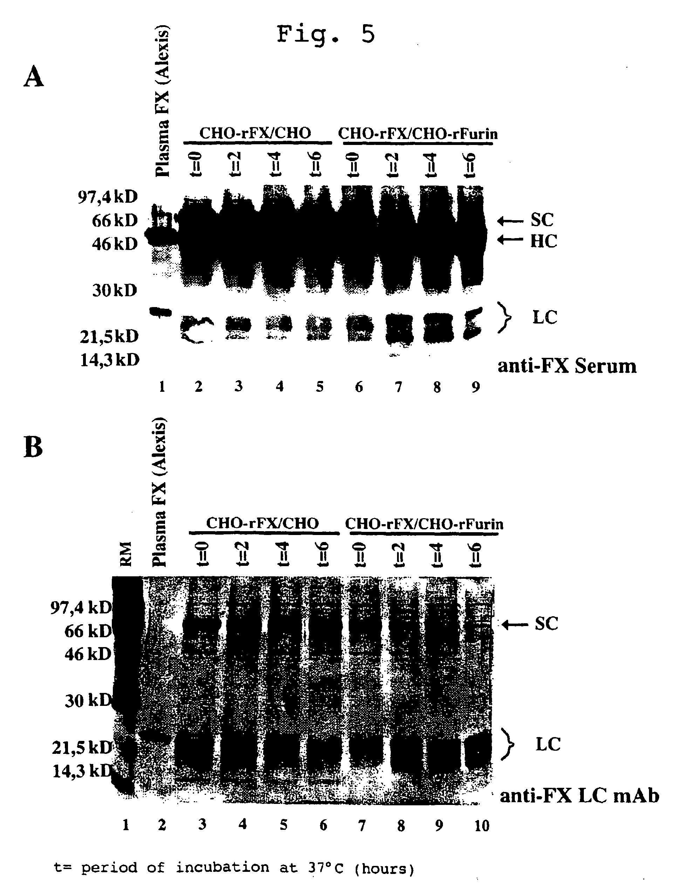

Zur Etablierung einer stabilen rFX-exprimierenden Zellinie wurden dhfr-defiziente CHO-Zellen mit dem Expressionsplasmid phAct-rFX und dem Selektionsmarkerplasmid pSV-dhfr co-transfiziert. Für alle weiteren Expressions- und Funktionsanalysen wurden die Zellkulturen mit serumfreiem Selektionsmedium in Anwesenheit von 10 µg/ml Vitamin K 24 Stunden lang inkubiert. Die Expression von rFX in den resultierenden Zellklonen wurde anhand der Antigenmenge (ELISA, Asserachrom, Boehringer Mannheim) nachgewiesen und das rekombinante Protein anschließend mit SDS-PAGE charakterisiert (Figur 4 A und B). In den Initialklonen und Subklonen davon liegt, wie im Western Blot erkennbar (Figur 4 A), das rekombinante FX-Protein in der Form einer leichten Kette (LC) von 22kD und einer schweren Kette (HC) von ca. 50 kD vor, die identisch mit dem plasmatischen Faktor X-Protein sind. Zusätzlich ist eine Proteinbande bei 75kD zu erkennen, die dem einzelkettigen (SC)-Molekül entspricht und deren Präsenz in FX-transfizierten CHO-Zellen (Wolf et al., J. Biol: Chem. 266:13726-13730, 1991) sowie in humanem Plasma (Fair et al., Blood 64:194-204, 1984) beschrieben wurde. Zur Herstellung von hochexprimierenden Klonen wurden die Initialklone mit steigenden Mengen Methotrexat amplifiziert und anschließend bis zur Stabilisierung subkloniert. Die Expression konnte von ca. 200-500 ng/10E6 Zellen bzw. 1 µg/ml auf 78 µg/10E6 Zellen bzw. 120 µg/ml pro 24 Stunden gesteigert werden. Die Western Blot-Analyse dieser hochexprimierenden Zellklonüberstände (Figur 4 B und Figur 5 A Spur 2) zeigt eine Anreicherung des einzelkettigen rFX-Moleküls sowie die Anwesenheit zusätzlicher Formen der leichten Kette. Neben der 22kD-Form der leichten Kette, die der plasmatischen Form entspricht (vollständig carboxyliert und ohne Propeptid), liegen drei weitere Varianten der leichten Kette mit ca. 21kD, 22,5kD und 20kD vor. Die Heterogenität der leichten Kette in diesen Klonen konnte mittels einer N-terminalen Sequenzierung des rekombinanten Materials auf eine unvollständige Abspaltung des Propeptids (hier: ca. 50% des rFX-Materials) sowie auf Untercarboxylierung (hier: ca. 50% des rFX) zurückgeführt werden. Das 21kD-Protein ist eine untercarboxylierte Propeptid-haltige und das 20kD-Protein eine untercarboxylierte Propeptid-freie Form der leichten Kette, während die 22,5 kD-Bande die vollständig carboxylierte, jedoch Pro-Peptid-haltige LC-Form repräsentiert.

### Beispiel 2 :

### Prozessierung von einzelkettigen rFX in rFX leichte/schwere Kette durch rFurin-Derivate

Aufgrund der Ähnlichkeit der Spaltstellen zwischen Faktor X Propeptid/N-Terminus der leichten Kette (RVTR↓A) und zwischen leichter/schwerer Kette (RRKR↓S) mit der Furin-Konsensus-Erkennungssequenz (RXK/RR↓X) bestand die Möglichkeit, die Prozessierung sowohl einzelkettiger als auch Propeptid-haltiger rFX-Moleküle durch rFurin-Derivate in vitro zu verbessern. In der Literatur werden für die beiden Prozessierungsschritte Proteasen vermutet, bei denen es sich jedoch nicht um Furin handelt (Rehemtulla et al., 1992, Blood 79:2349-2355; Wallin et al., 1994, Thromb. Res. 1994: 395-403).

Zellkulturüberstände von CHO-rFX und CHO-rFurin ΔTM6xHis (Patentanmeldung EP 0 775 750) sowie CHO-rFX und nicht-transfizierten CHO (als Negativkontrolle) wurden im Verhältnis 1:1 gemischt und bei 37°C inkubiert. Aliquote der Reaktionsansätze wurden vor Inkubation (t=0) und nach verschiedenen Inkubationszeiten (t=2, 4, 6 Stunden) mittels Western Blot-Analyse auf prozessierten rFX getestet (Figur 5). Der Nachweis von rFX in den Zellkulturüberständen erfolgte mittels eines anti-humanen FX-Antiserums (Figur 5 A) bzw. eines monoklonalen Antikörpers spezifisch für die leichte Kette des FX (Figur 5 B).

Im Gegensatz zu dem CHO-rFX/CHO-Gemisch weist das CHO-rFX/CHOrFurin schon nach zwei Stunden Inkubation bei 37°C (Figur 5 A Spur 7; Figur 5 B Spur 8) eine fast vollständige Prozessierung vor. Einzelkettiger rFX ist zum Großteil in die leichte und schwere Kettenform umgesetzt. Im Bereich der leichten Kette wurden nur noch die prozessierten Propeptid-freien Formen von 22kD (carboxylierte Form) und 20kD (untercarboxylierte Form) in einem Verhältnis von ca. 50:50 gefunden. Durch Optimieren der Zellkulturbedingungen kann dieses Verhältnis zugunsten der carboxylierten Form verbessert werden. Die korrekte Abspaltung der Pro-Sequenz zwischen Arg-1 und Ala+1 und die Homogenität des N-Terminus der leichten Kette wurde mittels N-terminaler Sequenzierung festgestellt. Im Kontrollexperiment, in dem CHO-rFX mit CHO-Überständen gemischt wurde, ist auch nach einer 6-stündigen Inkubation keine Veränderung des rFX-Bandenmusters zu erkennen (Fig. 5A, Spur 5; Figur 5B, Spur 6). Damit wurde nachgewiesen, daß rFurin im Überstand von CHO-Zellen biologisch aktiv ist und sowohl die Prozessierung des Propeptids als auch der schweren/leichten Kette von rFX durchführen kann.

### Beispiel 3 :

### Prozessierung von Faktor X mittels an Chelat-Tentakelgelimmobilisiertem rFurin

Um festzustellen, ob ein Substrat durch ein Säulen-gebundenes rFurin-Derivat gespalten werden kann, wurde untersucht, ob als Säulen-Matrix statt Ni²⁺-NTA-Agarose in einem experimentellen Ansatz Fractogel EMD® -Tentakelgel (Fa. Merck) verwendet werden kann. Da die Metallionen hierbei im Vergleich zur Ni²⁺-NTA-Agarose räumlich weiter von der eigentlichen Säulen-Matrix entfernt sind, könnte eine verbesserte sterische Zugänglichkeit des gebundenen rFurin-Derivats ermöglicht werden. In dem vorliegenden Ansatz wurde Pro-Faktor X durch Tentakelgel gebundenes rFurin-Derivat prozessiert:

Das Fractogel EMD® -Tentakelgel wurde nach Herstellervorschrift mit Ni²⁺-Ionen beladen und mit frischem Serum-freien Zellkulturmedium equilibriert. Anschliessend wurde die Säule mit Serumfreiem CHO-rFurin-Derivat-Überstand beladen. Waschschritte erfolgten durch Serum-freies Zellkulturmedium, enthaltend steigende Imidazol-Konzentrationen bis 40mM. Anschliessend wurde Pro-Faktor X als Serum-freier CHO-Überstand über die Säule geleitet. Mittels Western Blot-Analyse mit spezifischem Faktor X-Antiserum wurde die Prozessierung von Pro-Faktor X zu zweikettigem Faktor X im Durchfluß der Säule nachgewiesen.

### Beispiel 4 :

### Aktivität des in vitro prozessierten rekombinanten Faktor X

Rekombinanter Faktor X-Vorläufer wurde mit und ohne rFurin bei 4°C inkubiert. Zu verschiedenen Zeiten wurden Proben entnommen und bei -20°C weggefroren. Nach Abschluß der Inkubation (nach 4 Tagen) wurden alle Proben mittels FX-Coatest Kit (Fa. Chromogenix) auf FX-Aktivität getestet. Dazu wurden 50µl von jedem Überstand mit 50µl FX-defizientem, humanen Plasma versetzt und laut Protokoll des Herstellers rFX mit Schlangengift (RVV) in Anwesenheit von CaCl₂ zu rFXa umgesetzt; rFXa hydrolysiert anschließend das chromogene Substrat (S-2337) und führt zur Freisetzung des gelbfarbigen Paranitroanilins. Da die Menge an rFXa und die Farbintensität proportional zueinander sind, kann anhand einer Eichgerade, interpoliert aus Werten einer Plasma-Verdünnungsreihe, die Menge zu rFXa aktivierbarem rFX/ml-Zellkulturüberstand bestimmt werden. Mit diesen Ergebnissen und der bekannten rFX-Antigenmenge (ELISA-Daten) kann der Anteil von zu Faktor Xa aktiviertem rFaktor X in % ausgerechnet werden. Die Ergebnisse sind in Tabelle 1 dargestellt.

Um unspezifische, proteolytische Aktivität in CHO- und CHO-rFurin-Überständen auszuschließen, wurde das Gemisch dieser beiden Zellkulturüberstände ebenso untersucht.

CHO-rFX inkubiert mit CHO-Überständen (ohne rFurin) als Kontrolle zeigte auch nach 4 Tagen keine wesentliche Änderung der rFXa-Aktivität, die aufgrund der experimentellen Schwankungen bei etwa 800 mU/ml lag und 50 % - 60 % funktionellem rFX entsprach. Wurde im Vergleich dazu CHO-rFX mit CHO-rFurin inkubiert, so entstand während der Inkubationszeit eine konstante Steigerung der rFX-Aktivität, die von etwa 60 % (Zeitpunkt T=O) auf 86 % stieg (Tabelle 1). Damit wurde nachgewiesen, daß durch in vitro-Prozessierung von CHO-rFX aus hochexprimierenden Klonen mittels rFurin-Derivat der Anteil von zu funktionellem rFXa aktivierbaren rFX wesentlich verbessert wird.

**Tabelle 1**

| | Inkubation in Tagen | Aktivität in mU | Antigen Menge in µg/ml | Funktioneller Anteil an rFX in % |
|---|---|---|---|---|
| CHO-rFX+ CHO | 0 | 814 | 14 | 58 |
| | 1 | 847 | 14 | 61 |
| | 2 | 835 | 14 | 60 |
| | 3 | 790 | 14 | 56 |
| | 4 | 763 | 14 | 55 |
| CHO-rFX+ CHO-rFurin | 0 | 853 | 14 | 61 |
| | 1 | 1018 | 14 | 73 |
| | 2 | 1099 | 14 | 79 |
| | 3 | 1135 | 14 | 81 |
| | 4 | 1198 | 14 | 86 |
| CHO + CHO-rFurin | | 0 | | |
| Plasma FX 500mU | | 585 | | |

### Beispiel 5 :

### Expression von rekombinantem Faktor X in Furin-defizienten Zellen

Wie in den vorangegangenen Beispielen gezeigt, wird bei dem Faktor X-Vorläuferprotein sowohl die Propeptid-Abspaltung als die Spaltung der Einzelkette zu leichter/schwerer Kette in vitro durch Furin vermittelt. Dies legt nahe, daß diese Schritte auch in der Zelle endogen durch das ubiquitär vorkommende Furin, abhängig von der jeweilig exprimierten rFaktor X Menge mit unterschiedlicher Effizienz, bewerkstelligt werden. Dies wiederum führt zur Produktion einer Mischung heterogener rFaktor X-Formen.

Eine Möglichkeit, um eine möglichst homogene und zudem stabile Form von rFaktor X-Molekülen zu erzeugen, ist die Spaltung von rFaktor X durch endogene Proteasen, insbesondere Furin, zu unterbinden und somit funktionell inaktiven rFaktor X-Vorläufer (welche durch spätere Downstream-Prozessierung, idealerweise direkt vor Verwendung, in seine funktionell aktive Form umgewandelt werden kann) zu produzieren.

Dieses Verfahren wird speziell bei der Erzeugung von FX-Deletionsmutanten, die eine Furin-Spaltstelle anstatt der ursprünglichen Aktivierungsstelle enthalten, nützlich sein. Bei diesen Konstrukten kann die Aktivierung solch einer rekombinanten rFX-Mutante in vivo durch das endogene Furin stattfinden und zur Sekretion von aktivierten instabileren rFX-Formen führen. Die Degradation dieser Formen, durch CHO-Proteasen, z.B. in Zellkulturbedingungen mit hoher Zelllyse, während der Lagerung der Zellkulturüberstände oder dem Reinigungsverfahren, könnte zu inaktiven Abbauprodukten führen (Wolf et al., 1991).

Dieses Ziel kann z.B. durch Supplementieren des Zellkulturmediums mit Agenzien, die die intrazelluläre Furin-Aktivität reduzieren bzw. ausschalten kann, erreicht werden.

Eine andere Möglichkeit ist, a priori Furin-defiziente Zellen zu verwenden (Möhring et al., 1983, Infect. Immun. 41:998-1009; Ohnishi et al., 1994, J. Virol. 68:4075-4079; Gordon et al., 1995, Infect. Immun. 63:82-87).

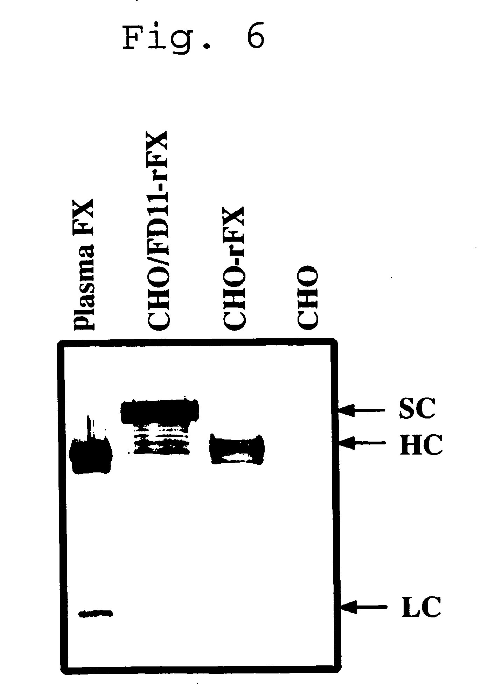

Hierfür wurde ein Furin-defizienter CHO-Zellklon FD11 (Gordon et al., 1995, Infect. Immun. 63:82-87) mit 20 µg phAct-FX und 1 µg pUCSV-neo (enthaltend das Neomycin-Resistenzgen im pUC-Vektor unter der Kontrolle des SV40-Promotors) co-transfiziert. Um stabile Klone zu erhalten, wurde das Medium mit 0,8 µg G418/ml supplementiert. Bei Vergleich sezernierter rFaktor X-Moleküle in serumfreien Überständen eines Furin-haltigen und eines Furin-defizienten CHO-Klons zeigt sich im Westernblot, daß in den Furin-defizienten Zellen rFaktor X-Vorläufer-Prozessierung unterbleibt und nur einzelkettiger Faktor X-Vorläufer vorliegt (Fig. 6); im Gegensatz dazu wird rFaktor X von "normalen" Zellen bei modester Expression noch vollständig, jedoch bei höherer Expression, trotz endogenem Furin, nur noch sehr beschränkt prozessiert. Aufgrund des geringen rFX-Expressionsgrades des verwendeten Zellklons ist die leichte Kette von rFaktor X im Blot hier nicht zu sehen.

### Beispiel 6:

### Herstellung von Faktor XΔ-Analogen (derzeit nach Ansicht der Anmelderin der beste Weg zur Ausführung der Erfindung)

### 6.1. Konstruktion von Expressionsplasmiden zur Herstellung von FX-Deletionsmutanten

Die Faktor X-Deletionsmutanten unterscheiden sich von der Faktor X-Wildtypsequenz durch die Deletion von den ca. 4,5 kDa großen Aktivierungspeptiden zwischen Aminosäure 180 bis 234). Zusätzlich wurden durch Mutagenese in dem C-Terminus der leichten Kette und/oder dem N-Terminus der schweren Kette verschiedene Spaltstellen eingebaut, die zur Aktivierung des daraus entstehenden einzelkettigen Faktor X-Moleküls zum aktivierten Polypeptid dienen. Die Expressionsplasmide für diese Faktor X-Deletionsmutanten sind alle von phAct-FX (beschrieben im Beispiel 1) abgeleitet.

Um die Klonierung der Faktor X-Deletionsmutanten zu vereinfachen, wurde das HindIII-NaeI DNA-Fragment aus Plasmid phAct-FX, das die Faktor X-kodierende Region von Position +1 bis +1116 umfaßt, in die HindIII/SmaI-Restriktionsschnittstellen von Plasmid pUC19 inseriert. Das resultierende Plasmid wurde mit pUC/FX bezeichnet. Zur Deletion des Aktivierungspeptids und Einbau von neuen Spaltstellen, z.B. Furin-, FXIa-, FXIIa, FXa-, FIIa-Spaltstellen, wurden das Bsp120I/BstXI FX-DNA-Fragment aus dem pUC/FX-Vektor durch synthetische Oligonucleotide ersetzt. Für den Einbau von einer Thrombin- oder FXIa-Spaltstelle wurde der BstXI-3'-Überhang mittels Mung Bean-Nuclease geglättet, so daß auch die Aminosäure Ile an Position 235 ausgetauscht werden konnte. Anschliessend wurden die deletierten Faktor X-DNA-Fragmente in Plasmid pAct-FX über HindIII-AgeI kloniert.

Zur Herstellung der Asp-Phe-Thr-Arg/Val FXIa-Spaltstelle wurde das Oligonucleotid sens #0009 (5'-GG CCC TAC CCC TGT GGG AAA CAG GAC TTC ACC AGG GTG-3') (SEQ. ID. Nr. 3) und das Oligonucleotid antisens #0010 (5'-CAC CCT GGT GAA GTC CTG TTT CCC ACA GGG GTA G-3') (SEQ. ID. Nr. 4) benutzt und in die Bsp120I- und die mit Mung Bean-Nuclease behandelte BstXI-Stelle eingesetzt. Dadurch wurden die Aminosäuren von Position 176 bis 178 und 235 in Asp-Phe-Thr und Val mutiert (Fig. 2 A).

Zur Herstellung der Arg/Thr FIIa-Spaltstelle wurde das Oligonucleotid sens #0011 (5 '-GG CCC TAC CCC TGT GGG AAA CAG ACC CTG GAA CGG ACC-3') (SEQ. ID. Nr. 5) und das Oligonucleotid antisens #0012 (5'-GGT CCG TTC CAG GGT CTG TTT CCC ACA GGG GTA G-3') (SEQ. ID. Nr. 6) benutzt und in die Bsp120I- und die mit Mung Bean-Nuclease behandelte BstXI-Stelle eingesetzt. Dadurch wurde die Aminosäure Ile an Position 235 in Thr mutiert (Fig. 2 B).

Zur Herstellung der Ile-Lys-Pro-Arg/Ile FXIIa-Spatstelle wurde das Oligonucleotid sens #0013 (5'-GG CCC TAC CCC TGT GGG AAA CAG ATC AAG CCC AGG ATC-3') (SEQ. ID. Nr. 7) und das Oligonucleotid antisens #0014 (5'-CT GGG CTT GAT CTG TTT CCC ACA GGG GTA G-3') (SEQ. ID. Nr. 8) benutzt und in die Bsp120I- und BstXI-Stellen eingesetzt. Dadurch wurden die Aminosäuren von Position 176 bis 178 in Ile-Lys-Pro mutiert (Fig. 2 C).

Zur Herstellung der Ser-Met-Thr-Arg/Ile Kallikrein-Spaltstelle wurde das Oligonucleotid sens #0015 (5'-GG CCC TAC CCC TGT GGG AAA CAG AGC ATG ACC AGG ATC-3') (SEQ. ID. Nr. 9) und das Oligonucleotid #0016 (5'-CT GGT CAT GCT CTG TTT CCC ACA GGG GTA G-3') (SEQ. ID. Nr. 10) benutzt und in die Bsp120I-und BstXI-Stellen eingesetzt. Dadurch wurden die Aminosäuren von Position 176 bis 178 in Ser-Met-Thr mutiert (Fig. 2 D).

Zur Herstellung einer Met-Lys-Thr-Arg/Ile FXa-Spaltstelle wurde das Oligonucleotid sens #0033 (5'-GG CCC TAC CCC TGT GGG AAA CAG ATG AAA ACG AGG ATC-3') (SEQ. ID. Nr. 11) und das Oligonucleotid antisens #0034 (5'-CT CGT TTT CAT CTG TTT CCC ACA GGG GTA G-3') (SEQ. ID. Nr. 12) benutzt und in die Bsp120I- und BstXI-Stellen eingesetzt. Dadurch wurden die Aminosäuren in Position 176 bis 178 von Thr-Leu-Glu in Met-Lys-Thr mutiert (Fig. 2 E).

Zur Herstellung einer Ile-Glu-Gly-Arg/Ile FXa-Spaltstelle wurde das Oligonucleotid sens #0035 (5'-GG CCC TAC CCC TGT GGG AAA CAG ATC GAG GGA AGG ATC-3') (SEQ. ID. Nr. 13) und das Oligonucleotid antisens #0036 (5'-CT TCC CTC GAT CTG TTT CCC ACA GGG GTA G-3') (SEQ. ID. Nr. 14) benutzt und in die Bsp120I- und BstXI-Stellen eingesetzt. Dadurch wurden die Aminosäuren in Position 176 bis 178 von Thr-Leu-Glu in Ile-Glu-Gly mutiert (Fig. 2 F).

Zur Herstellung einer Arg-Arg-Lys-Arg/Ile Furin-Spaltstelle wurde das Oligonucleotid sens #0017 (5'-GG CCC TAC CCC TGT GGG AAA CAG AGG AGG AAG AGG ATC-3') (SEQ. ID. Nr. 15) und das Oligonucleotid antisens #0018 (5'-CT CTT CCT CCT CTG TTT CCC ACA GGG GTA G-3') (SEQ. ID. Nr. 16) benutzt und in die Bsp120I- und BstXI-Stellen eingesetzt. Dadurch wurden die Aminosäuren von Position 176 bis 178 in Arg-Arg-Lys mutiert (Fig. 2 G).

Zur Herstellung einer Arg-Val-Arg-Arg/Ile Furin-Spaltstelle wurde das Oligonucleotid sens #0019 (5'-GG CCC TAC CCC TGT GGG AAA CAG AGG GTG AGG AGG ATC-3') (SEQ. ID. Nr. 17) und das Oligonucleotid antisens #0020 (5'-CT CCT CAC CCT CTG TTT CCC ACA GGG GTA G-3') (SEQ. ID. Nr. 18) benutzt und in die Bsp120I- und BstXI-Stellen eingesetzt. Dadurch wurden die Aminosäuren von Position 176 bis 178 in Arg-Val-Arg mutiert (Fig. 2 G).

Zur Herstellung einer Arg-Arg-Arg-Arg/Ile Furin-Spaltstelle wurde das Oligonucleotid sens #0021 (5'-GG CCC TAC CCC TGT GGG AAA CAG AGG AGG AGG AGG ATC-3') (SEQ. ID. Nr. 19) und das Oligonucleotid antisens #0022 (5'-CT CCT CCT CCT CTG TTT CCC ACA GGG GTA G-3') (SEQ. ID. Nr. 20) benutzt und in die Bsp120I- und BstXI-Stellen eingesetzt. Dadurch wurden die Aminosäuren von Position 176 bis 178 in Arg-Arg-Arg mutiert (Fig. 2 G).

Zur Herstellung einer Arg-Pro-Lys-Arg/Ile Furin-Spaltstelle wurde das Oligonucleotid sens #0023 (5'-GG CCC TAC CCC TGT GGG AAA CAG AGG CCC AAG AGG ATC-3') (SEQ. ID. Nr. 21) und das Oligonucleotid antisens #0024 (5'-CT CTT GGG CCT CTG TTT CCC ACA GGG GTA G-3') (SEQ. ID. Nr. 22) benutzt und in die Bsp120I- und BstXI-Stellen eingesetzt. Dadurch wurden die Aminosäuren von Position 176 bis 178 in Arg-Pro-Lys mutiert (Fig. 2 G).

Zur Herstellung einer Ile Arg-Lys-Arg/Ile Furin-Spaltstelle wurde das Oligonucleotid sens #0025 (5'-GG CCC TAC CCC TGT GGG AAA CAG ATC AGG AAG AGG ATC-3') (SEQ. ID. Nr. 23) und das Oligönucleotid antisens #0026 (5'-CT CTT CCT GAT CTG TTT CCC ACA GGG GTA G-3') (SEQ. ID. Nr. 24) benutzt und in die Bsp120I- und BstXI-Stellen eingesetzt. Dadurch wurden die Aminosäuren von Position 176 bis 178 in Ile-Arg-Lys mutiert (Fig. 2 G).

Zur Herstellung einer Arg-Ser-Lys-Arg/Ile Furin-Spaltstelle wurde das Oligonucleotid sens #0027 (5'-GG CCC TAC CCC TGT GGG AAA CAG AGG AGC AAG AGG ATC-3') (SEQ. ID. Nr. 25) und das Oligonucleotid antisens #0028 (5'-CT CTT GCT CCT CTG TTT CCC ACA GGG GTA G-3') (SEQ. ID. Nr. 26) benutzt und in die Bsp120I- und BstXI-Stellen eingesetzt. Dadurch wurden die Aminosäuren von Position 176 bis 178 in Arg-Ser-Lys mutiert (Fig. 2 G).

Zur Herstellung einer Arg-Val-Thr-Arg/Ile Furin-Spaltstelle wurde das Oligonucleotid sens #0029 (5'-GG CCC TAC CCC TGT GGG AAA CAG AGG GTC ACG AGG ATC-3') (SEQ. ID. Nr. 27) und das Oligonucleotid antisens #0030 (5'-CT CGT GAC CCT CTG TTT CCC ACA GGG GTA G-3') (SEQ. ID. Nr. 28) benutzt und in die Bsp120I- und BstXI-Stellen eingesetzt. Dadurch wurden die Aminosäuren von Position 176 bis 178 in Arg-Val-Thr mutiert (Fig. 2 G).

Zur Herstellung einer Arg-Leu-Lys-Arg/Ile Furin-Spaltstelle wurde das Oligonucleotid sense #0031 (5'-GG CCC TAC CCC TGT GGG AAA CAG AGG CTG AAA AGG ATC-3') (SEQ. ID. Nr. 29) und das Oligonucleotid antisense #0032 (5'-CT TTT CAG CCT CTG TTT CCC ACA GGG GTA G-3') (SEQ. ID. Nr. 30) benutzt und in die Bsp120I- und BstXI-Stellen eingesetzt. Dadurch wurden die Aminosäuren von Position 176 und 178 in Arg und Lys mutiert (Fig. 2 G).

Zur Herstellung einer Pro-Gln-Gly-Arg/Ile FXa-Spaltstelle wurde das Oligonucleotid sens #0037 (5'-GG CCC TAC CCC TGT GGG AAA CAG CCC CAA GGA AGG ATC-3') (SEQ. ID. Nr. 31) und das Oligonucleotid antisens #0038 (5'-CT TCC TTG GGG CTG TTT CCC ACA GGG GTA G-3') (SEQ. ID. Nr. 32) benutzt und in die Bsp120I- und BstXI-Stellen eingesetzt. Dadurch wurden die Aminosäuren in Position 176 bis 178 von Thr-Leu-Glu in Pro-Gln-Gly mutiert (Fig. 2 H).

Zur Herstellung der Thr-Ser-Thr-Arg/Ile FXIIa-Spaltstelle wurde das Oligonucleotid sens #0039 (5'-GG CCC TAC CCC TGT GGG AAA CAG ACG AGC ACG AGG ATC-3') (SEQ. ID. Nr. 33) und das Oligonucleotid antisens #0040 (5'-CT CGT GCT CGT CTG TTT CCC ACA GGG GTA G-3') (SEQ. ID. Nr. 34) benutzt und in die Bsp120I- und BstXI-Stellen eingesetzt. Dadurch wurden die Aminosäuren von Position 176 und 178 in Ser-Thr mutiert (Fig. 2 I).

Zur Herstellung einer Arg/Ile Trypsin-Spaltstelle wurde das Oligonucleotid #0041 (5'-GG CCC TAC CCC TGT GGG AAA CAG ACC CTG GAA CGG ATC-3') (SEQ. ID. Nr. 35) und das Oligonucleotid antisens #0042 (5'-CG TTC CAG GGT CTG TTT CCC ACA GGG GTA G-3') (SEQ. ID. Nr. 36) benutzt und in die Bsp120I- und BstXI-Stellen eingesetzt (Fig. 2 J).

Die resultierenden Expressionsplasmide (siehe Fig. 3) umfassen den humanen beta-Actin-Promoter, 78bp des 5'UTR, das beta-Actin-Intron, die modifizierte Faktor X-Sequenz sowie 39bp der 3'UTR und die SV40-Polyadenylierungsstelle.

### 6.2. Konstruktion von Expressionsplasmiden für die Herstellung von FXβ-Analogon.

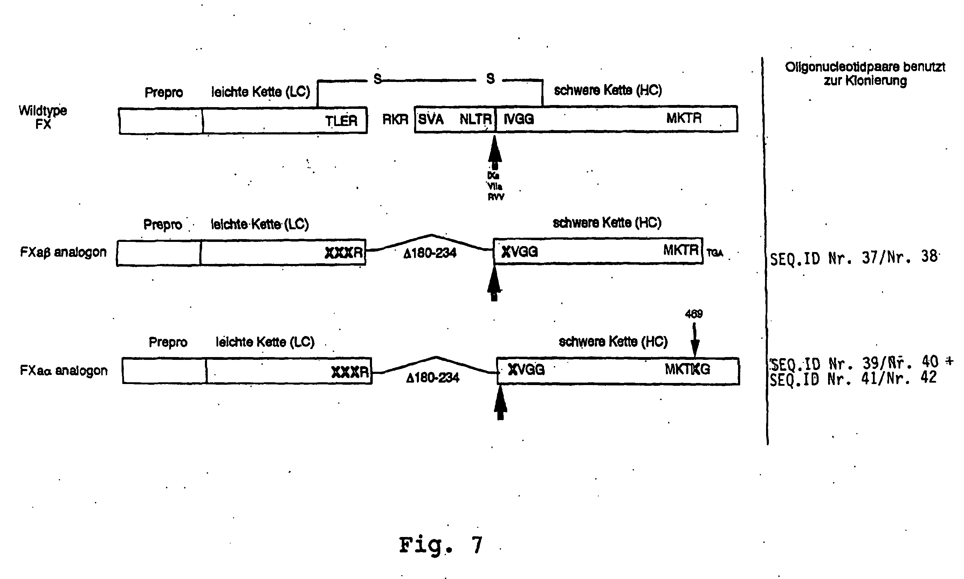

Diese Konstrukte wurden von den oben beschriebenen Faktor XΔ-Analogon-Konstrukten abgeleitet, indem ein TGA-Stopcodon an Position 470 eingebaut wurde. Dazu wurden die Aminosäuren von Position 457 bis zum Stopcodon durch SpeI und partiellen BstEII-Verdau entfernt und mit dem Oligonucleotidpaar #0003 (5'-GTC ACC GCC TTC CTC AAG TGG ATC GAC AGG TCC ATG AAA ACC AGG TGA A-3') (SEQ. ID. Nr. 37) und #0004 (5'-CTA GTT CAC CTG GTT TTC ATG GAC CTG TCG ATC CAC TTG AGG AAG GCG-3') (SEQ. ID. Nr. 38) ersetzt. Eine schematische Darstellung der Faktor XΔβ-Analogon-Konstrukte ist in Fig. 7 gezeigt. Zur Vereinfachung der Abbildung wurden alle Faktor XΔβ-Analoge als ein allgemeines Konstrukt dargestellt, in dem die variable Aminosäuren in der Spaltstellenregion als schattiertes "X" angegeben sind.

### 6.3. Konstruktion von Expressionsplasmiden für die Herstellung von FXΔα-Analogon

Mit der Aktivierung des Faktor X durch die Abspaltung des 4,5 kDa-Aktivierungspeptids am N-terminalen Ende der schweren Kette entsteht die Faktor Xaα-Form. Diese Form wird anschließend durch auto-proteolytische Aktivität und Abspaltung des C-Terminus der schweren Kette zwischen Arg 469 und Gly 470 in die FXaβ-Form umgesetzt. Zur Herstellung von Faktor X-Expressionsplasmiden, die zur Produktion von Faktor XΔ-Analogen führen, die nach Aktivierung ausschließlich als FXaα-Form mit intaktem β-Peptid vorliegen, wurde die Aminosäure Arg469 in Lys mutiert, sodaß keine Prozessierung im C-terminalen Bereich der schweren Kette mehr stattfinden kann.

Dazu wurde die für die C-terminale Aminosäuresequenz codierende DNA-Sequenz des Faktor X von Position 1363 bis zum Stopsignal durch partiellen BstEII-SpeI- Verdau entfernt und durch zwei zusammenligierte Oligonucleotidpaare ersetzt. Oligonucleotide #0005 (5'-GTC ACC GCC TTC CTC AAG TGG ATC GAC AGG TCC ATG AAA ACC AAG GGC TTG CCC AAG-3') (SEQ. ID. Nr. 39) und Oligonucleotid #0006 (5'-TTG GCC TTG GGC AAG CCC TTG GTT TTC ATG GAC CTG TCG ATC CAC TTG AGG AAG GCG-3') (SEQ. ID. Nr. 40) wurden mit Oligonucleotid #0007 (5'-GCC AAG AGC CAT GCC CCG GAG GTC ATA ACG TCC TCT CCA TTA AAG TGA GAT CCC A-3') (SEQ. ID. Nr. 41) und Oligonucleotid #0008 (5'-CTA GTG GGA TCT CAC TTT AAT GGA GAG GAC GTT ATG ACC TCC GGG GCA TGG CTC-3') (SEQ. ID. Nr. 42) ligiert. Die Mutation der Aminosäure Arg469 wird durch das Oligonucleotidpaar #0005-#0006 eingeführt. Eine schematische Darstellung der FXΔ-Analoge ist in Figur 7 gezeigt.

### Beispiel 7:

### Bestimmung der N-Termini von Faktor X und Prozeseierungsprodukten mit und ohne rFurin

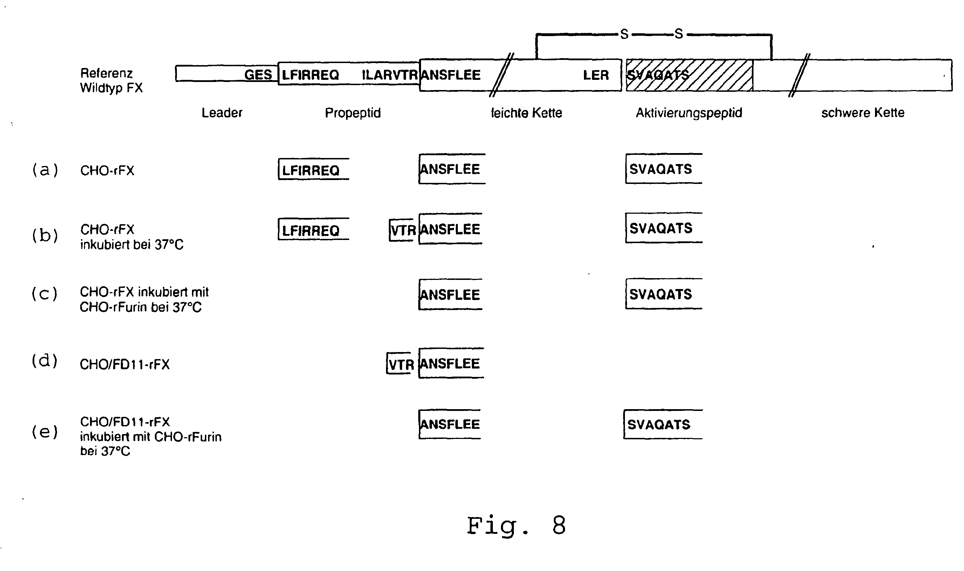

Rekombinanter Faktor X wurde, wie in Beispiel 1 beschrieben, in CHO-Zellen mit endogenem Furin bzw., wie in Beispiel 5 beschrieben, in Furin-defizienten Zellen exprimiert. rFaktor X wurde sowohl aus a) nicht vorbehandeltem, b) 12 Stunden bei 37°C inkubiertem und c) 12 Stunden bei 37°C mit CHO-rFurin-Überstand vorbehandeltem Zellkulturüberstand hochexprimierender CHO-rFX-Klone, als auch aus d) nicht vorbehandeltem und e) 12 Stunden bei 37°C mit CHO-rFurin-Überstand vorbehandeltem Zellkulturüberstand von CHO-FD11-rFX-Klone isoliert. Die endständigen N-terminalen Aminosäuren von Faktor X und Prozessierungsprodukten der einzelnen Reaktionsansätze a) bis e) wurden über Edman-Analyse bestimmt. Figur 8 zeigt eine schematische Darstellung der Ergebnisse.

Der rFaktor X aus hochexprimierenden CHO-Zellen tritt in Form der reifen schweren und leichten Ketten, sowie einzelkettig, zum Teil noch Propeptid-haltig auf. Nach einer Inkubation dieser Zellkulturüberstande für 12 Stunden bei 37°C (b) treten zusätzlich, wie schon von Wolf et al. (1991, J. Bio. Chem. 266:13726-13730) beschrieben, fehlerhafte N-Termini der rFX-leichten Kette mit 3 zusätzlichen Aminosäuren Va138-Thr39-Arg40 auf. Diese kryptischen Enden werden auch bei der Sequenzierung von rFX-Material aus nicht vorbehandelten CHO-FD11-Zellen (d) gefunden. Diese Beobachtung zeigt, daß das Auftreten von diesen fehlerhaften N-Termini vermieden werden kann, indem angemessene Bedingungen bzw. Zellkulturbedingungen, Lagerung und Reinigungsverfahren zur Minimierung der rFX-Proteolyse durch CHO-Proteasen, benützt werden.

Im Gegensatz zu dem gereinigten Material aus CHO-Zellen (a und b) ist der rFX aus nicht amplifizierten, Furin-defizienten Zellen (d) nur in Form unprozessierter einzelkettiger Vorläufer vorhanden. Es werden auch keine N-terminalen Sequenzen gefunden, die dem Propeptid-Anteil entsprechen. Damit wurde gezeigt, daß die Prozessierung von einzelkettigem rFX-Vorläufer in leichte/schwere Kette in Furin-defizienten CHO-Zellen (d) nicht mehr stattfindet, was auf eine zentrale Rolle der Endoprotease Furin in diesem Prozessierungschritt in vivo schliessen läßt. Zusätzlich wurde gezeigt, daß die Prozessierung Propeptid-haltiger rFX-Moleküle auch in Furin-defizienten CHO-Zellen stattfindet und daher Furin in vivo keine essentielle Rolle in diesem Prozessierungsschritt spielt. Durch die Inkubation von rFX aus CHO-Zellen (c) und CHO-FD11-Zellen (e) in Gegenwart von Furin werden ausschließlich leichte und schwere Ketten mit korrekten N-Termini gefunden. Damit wurde nachgewiesen, daß sowohl die einzelkettigen FX-Vorläufer als auch die Propeptid-haltigen rFX-Moleküle, durch in vitro-Prozessierung in homogenen, reifen Faktor X umgesetzt werden. Damit weist der in Gegenwart von Furin prozessierte Faktor X eine aussergewöhnliche strukturelle Integrität auf.

### Beispiel 8:

### Expression und Charakterisierung der rekombinanten FX- Deletionsmutante mit der Spaltstelle Arg-Val-Thr-Arg/Ile (FXΔ^{RVTR/I})

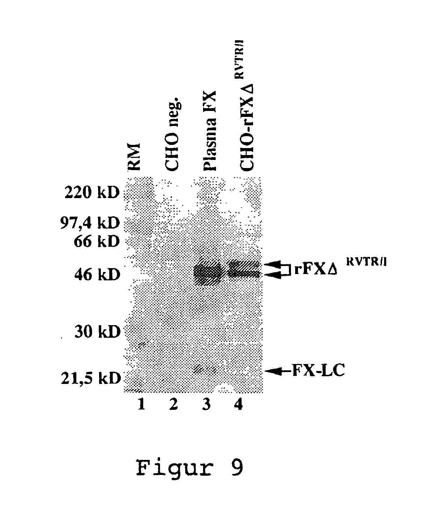

Das Expressionsplasmid, das für die FX-Deletionsmutante mit der Spaltstelle Arg-Val-Thr-Arg/Ile (FXΔ^{RVTR/I}) kodiert, wurde, wie schon in Beispiel 1 beschrieben, mit dem Selektionsmarker pSV/dhfr in dhfr-defiziente CHO-Zellen co-transfiziert. Das rekombinante Protein FXΔ^{RVTR/I} aus permanenten CHO-Klonen wurde mittels Western Blot-Analyse charakterisiert. Wie in Figur 9, Spur 4 sichtbar ist, tritt das rekombinante Protein in der Form einer Doppelbande von ca. 56 und 50kD auf. Im Zellkulturüberstand nicht transfizierter CHO-Zellen ist kein FX-reaktives Material detektierbar (Spur 2). Diese Ergebnisse schließen aus, daß diese Proteinbanden aufgrund einer Verunreinigung der analysierten Überstände von Wildtyp FX aus Resten an bovinem Serum im Zellkulturmedium auftreten. Es ist deshalb wahrscheinlich, daß die Doppelbande auf unterschiedliche posttranslationale Modifikationen, z.B. die Anwesenheit des Propeptids oder unterschiedliche Glycosylierung des rFXΔ^{RVTR/I}-Moleküls, zurückzuführen ist.

Die in diesem Konstrukt eingefügte Spaltstelle Arg-Val-Thr-Arg/Ile ist identisch mit der Propeptid-Spaltstelle des Wildtyp FX-Moleküls, die durch eine CHO-Endoprotease in vivo effizient erkannt und gespalten wird (siehe Beispiel 7). Die Western Blot-Analyse zeigt keine zusätzlichen 35kD- bzw. 31kD-schweren FX-Moleküle, die den aktivierten α- und β-Formen der rFXΔ^{RVTR/I-}schweren Ketten entsprechen würden. Diese Ergebnisse zeigen, daß entweder die Menge an Endoprotease für die Aktivierung des Proteins nicht ausreichend ist oder/und, daß die Spaltstelle Arg-Val-Thr-Arg/Ile in der vorliegenden Sequenzumgebung in vivo nicht oder nur ineffizient erkannt und gespalten wird. rFXΔ^{RVTR/I} liegt demnach praktisch ausschließlich einkettig vor.

### Beispiel 9:

### Aktivierung des rekombinanten rFXΔ^{RVTR/I}-Proteins mittels rekombinanter Furin-Derivate in vitro.

Obwohl die Spaltstelle Arg-Val-Thr-Arg im rFX-Propeptid in vivo von einer anderen Protease als Furin erkannt wird, wurde im Beispiel 2 bewiesen, daß diese Sequenz sehr effizient und korrekt durch ein rFurin-Derivat in vitro gespalten wird.

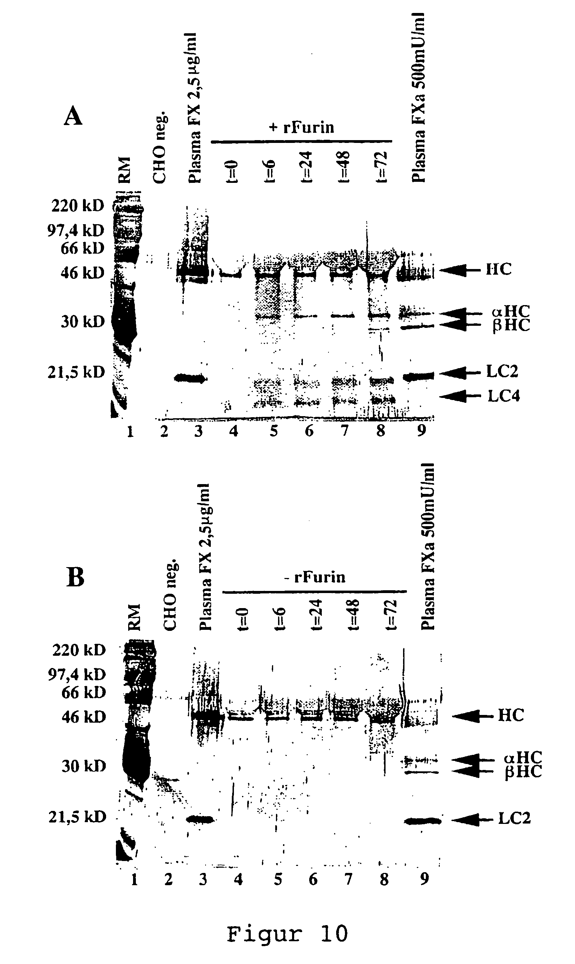

Um die Aktivierbarkeit des rFXΔ^{RVTR/I}-Proteins durch rFurin in vitro zu testen, wurden Mischexperimente durchgeführt. Hierfür wurde Zellkulturüberstand von CHO-FXΔ^{RVTR/I}-Zellen mit gereinigtem rFurin-Derivat rFurinΔCys-Spacer-10xHis (siehe Patentanmeldung EP 0 775 750 A2) in Anwesenheit von 20mM Hepes, pH 7, 0, 150 mM NaCl, 4mM CaCl₂ und 0,1% BSA in einem 1:1-Verhältnis versetzt. Im Kontrollexperiment wurden der CHO-rFXΔ^{RVTR/I}-Überstand im gleichen Verhältnis nur mit dem BSA-haltigen Puffer gemischt. Die Zugabe von BSA soll die enzymatische Aktivität des rFurin-Derivates sowie die in Folge entstehenden aktivierten rFXΔ^{RVTR/I}-Produkte stabilisieren. Aliquots der Reaktionsansätze vor und nach einer Inkubationszeit von 6, 24, 48 und 72 Stunden (t=0, t=6, t=24, t=48, t=72) bei 37°C wurden auf rFXΔ^{RVTR/I-}Prozessierung mittels Western Blot-Analyse getestet (Figur 10). Im Mischexperiment ohne rFurin-Zugabe (Fig. 10B) ist keine Veränderung des Bandenmusters im Laufe der Inkubationszeit sichtbar (Spur 4 bis 9). Aufgrund der Präsenz des BSA in den Reaktionsansätzen sind nur die leichteren rFXΔ^{RVTR/I}-Moleküle (50 kD) gut sichtbar, weil die 56kD-schweren Moleküle von der BSA-Bande überdeckt werden.

In Anwesenheit des rFurin-Derivates (Fig. 10 A) tritt schon nach 6 Stunden Inkubation (Spur 5) eine 35kD große Proteinbande auf, die der α-Form der FX-schweren Kette entspricht (Vergleich mit Spur 9) auf. Dieses Protein akkumuliert im Laufe der Inkubation und wird anschließend auch, wie bei Plasma FX bekannt, in die proteolytische β-Form umgesetzt, welche durch proteolytische Umwandlung aus der α-Form entsteht (Spur 7 und 8). Parallel zur Detektion der aktivierten Formen der schweren Ketten werden leichte Ketten von 22kD und 20kD sichtbar, die in Beispiel 1.b. als Propeptid-freie, carboxylierte LC2 (die der eigentlich funktionellen Form entspricht) bzw. als Propeptid-freie, untercarboxylierte LC4-Form der leichten Kette identifiziert wurden. Die Anwesenheit der untercarboxylierten LC4-Form bestätigt, daß in den analysierten CHO-Klonen die posttranslationalen Modifikationsmechanismen limitiert sind. Obwohl die 50kD-Bande unverändert erscheint, während scheinbar die 56kD-Form direkt zu leichten/schweren Ketten abgebaut wird, so wird tatsächlich das 56kD-Molekül zunächst zur 50kD-Form umgewandelt und erst anschließend in eine leichte und eine schwere Kette gespalten. Dies ist auf die Anwesenheit des Propeptids im 56kD-Molekül zurückzuführen, welches zunächst unter Bildung der 50kD-Form entfernt wird.

Damit wurde gezeigt, daß das rFXΔ^{RVTR/I}-Konstrukt über die eingebaute Arg-Val-Thr-Arg/Ile-Spaltstelle durch rFurin-Derivate in vitro aktivierbar ist, und die entstehenden Prozessierungsprodukte des rFXΔ^{RVTR/I}-Konstrukts in der Größe jenen von Plasma FXa entsprechen. Das Auftreten von FXΔβ, die durch autoproteolytische Prozessierung von FXΔα entsteht, zeigt die Funktionalität des rFXΔ^{RVTR/I}-Moleküls.

## Patentansprüche

1. Faktor XΔ-Analogon, **dadurch gekennzeichnet, dass** es eine Deletion der Aminosäuren Arg180 bis Arg234 der Faktor XΔ-Aminosäuresequenz und eine Modifikation im Bereich der Aminosäuresequenz zwischen Gly173 und Arg179 aufweist, wobei die Modifikation eine Prozessierungsstelle einer nicht-natürlicherweise an dieser Stelle der Faktor X-Sequenz spaltenden Protease darstellt.

2. Faktor XΔ-Analogon nach Anspruch 1, **dadurch gekennzeichnet, dass** die Modifikation mindestens ein Aminosäureaustausch im Bereich der Aminosäuresequenz zwischen Gly173 und Arg179 bezogen auf die Aminosäurenumerierung gemäß Fig. 1, ist.

3. Faktor XΔ-Analogon nach Anspruch 1 oder 2, **dadurch gekennzeichnet, dass** es eine Faktor X-Sequenz mit Gly173-R6-R5-R4-R3-R2-Arg179/R1(235) enthält, wobei
a) R1 eine Aminosäure ausgewählt aus der Gruppe Val, Ser, Thr, Ile oder Ala
b) R2 eine Aminosäure ausgewählt aus der Gruppe Glu, Thr, Pro, Gly, Lys oder Arg
c) R3 eine Aminosäure ausgewählt aus der Gruppe Leu, Phe, Lys, Met, Gln, Glu, Ser, Val, Arg oder Pro
d) R4 eine Aminosäure ausgewählt aus der Gruppe Thr, Asp, Asn, Ile, Ser, Met, Pro, Arg oder Lys
e) R5 eine Aminosäure ausgewählt aus der Gruppe Asn, Lys, Ser, Glu, Gln, Ala, His oder Arg und
f) R6 eine Aminosäure ausgewählt aus der Gruppe Asp, Phe, Thr, Arg, Leu oder Ser ist.

4. Faktor XΔ-Analogon nach einem der Ansprüche 1 bis 3, **dadurch gekennzeichnet, dass** die Modifikation eine Prozessierungsstelle für eine Protease ausgewählt aus der Gruppe der Endoproteasen, wie etwa Kexin/Kex2, Furin/PACE, PC1/PC3, PC2, PC4, PACE 4, LPC/PC7, der Serinproteasen, wie etwa Faktor IIa, Faktor VIIa, Faktor IXa, Faktor XIIa, Faktor XIa, Faktor Xa, oder von Kallikrein, oder einem Derivat dieser Proteasen, darstellt.

5. Faktor XΔ-Analogon nach einem der Ansprüche 1 bis 4, **dadurch gekennzeichnet, dass** es als einzelkettiges Molekül in enzymatisch inaktiver Form vorliegt.

6. Faktor XΔ-Analogon nach einem der Ansprüche 1 bis 5, **dadurch gekennzeichnet, dass** die Modifikation eine Aktivierung des inaktiven, einzelkettigen Faktor XΔ-Analogon-Polypeptides in die zweikettige, aktive Faktor Xa-Analogon-Form erlaubt.

7. Faktor XΔ-Analogon nach einem der Ansprüche 1 bis 6, **dadurch gekennzeichnet, dass** es eine weitere Modifikation im Bereich der C-terminalen Faktor X-Aminosäuresequenz aufweist.

8. Faktor XΔ-Analogon nach Anspruch 7, **dadurch gekennzeichnet, dass** es eine Modifikation im C-terminalen Bereich der β-Peptidspaltstelle aufweist.

9. Faktor XΔ-Analogon nach Anspruch 8, **dadurch gekennzeichnet, dass** die Modifikation eine Mutation, Deletion oder Insertion im Bereich der Faktor X-Aminosäuresequenz zwischen Aminosäureposition Arg469 und Ser476 ist.

10. Faktor XΔ-Analogon nach einem der Ansprüche 7 bis 9, **dadurch gekennzeichnet, dass** die Modifikation eine Abspaltung des β-Peptids verhindert.

11. Faktor XΔ-Analogon nach Anspruch 7, **dadurch gekennzeichnet, dass** es eine Deletion des Faktor Xβ-Peptids aufweist.

12. Faktor XΔ-Analogon nach Anspruch 11, **dadurch gekennzeichnet, dass** es ein Translationsstopsignal im C-terminalen Bereich der Faktor X-Sequenz aufweist.

13. Faktor XΔ-Analogon nach Anspruch 12, **dadurch gekennzeichnet, dass** es ein Translationsstopsignal an Position der Aminosäure Lys470 der Faktor X-Sequenz aufweist.

14. Faktor XΔ-Analogon nach einem der Ansprüche 1 bis 13, **dadurch gekennzeichnet, dass** die Modifikation im Bereich der Aminosäuresequenz zwischen Gly173 und Arg179 eine Aktivierung des inaktiven Faktor X-Analogons zu aktivem Faktor XΔ-Analogon in vitro erlaubt.

15. Faktor XΔ-Analogon nach Anspruch 14, **dadurch gekennzeichnet, dass** die Modifikation eine Aktivierung durch eine Protease, ausgewählt aus der Gruppe der Endoproteasen, wie etwa Kexin/Kex2, Furin/PACE, PC1/PC3, PC2, PC4, PACE 4, LPC/PC7, der Serinproteasen, wie etwa Faktor IIa, Faktor VIIa, Faktor Ixa, Faktor XIIa, Faktor XIa, Faktor Xa oder von Kallikrein, oder einem Derivat dieser Proteasen, erlaubt.

16. Faktor XΔ-Analogon nach einem der Ansprüche 1 bis 15, **dadurch gekennzeichnet, dass** es ein intaktes β-Peptid aufweist und als Faktor XΔα vorliegt.

17. Faktor XΔ-Analogon nach einem der Ansprüche 1 bis 15, **dadurch gekennzeichnet, dass** es eine Deletion des β-Peptids aufweist.

18. Rekombinante DNA, kodierend für ein Faktor XΔ-Analogon nach einem der Ansprüche 1 bis 17, enthalten in einem Vektor zur rekombinanten Expression des kodierten Proteins.

19. Transformierte Zellen enthaltend eine rekombinante DNA gemäß Anspruch 18.

20. Präparation enthaltend gereinigtes Faktor XΔ-Analogon, das eine Deletion der Aminosäuren Arg180 bis ARG234 der Faktor X-Aminosäuresequenz und eine Modifikation im Bereich der Aminosäuresequenz zwischen Gly173 und Arg179 aufweist, wobei die Modifikation eine Prozessierungsstelle einer nicht-natürlicherweise an dieser Stelle der Faktor X-Sequenz spaltenden Protease darstellt.

21. Präparation nach Anspruch 20, **dadurch gekennzeichnet, dass** sie ein einzelkettiges Faktor XΔ-Analogon in enzymatisch inaktiver Form mit einer Reinheit von mindestens 80%, vorzugsweise 90%, besonders bevorzugt 95%, enthält und keine inaktiven, proteolytischen Intermediate von Faktor X/Xa-Analogon aufweist.

22. Präparation nach einem der Ansprüche 20 oder 21, **dadurch gekennzeichnet, dass** sie Faktor XΔ-Analogon als Faktor XΔα enthält.

23. Präparation nach einem der Ansprüche 20 oder 21, **dadurch gekennzeichnet, dass** sie Faktor XΔ-Analogon als FXΔβ enthält.

24. Präparation nach einem der Ansprüche 20 bis 23, **dadurch gekennzeichnet, dass** sie Faktor XΔ-Analogon als einzelkettiges Molekül in isolierter Form enthält.

25. Präparation nach einem der Ansprüche 20 bis 24, **dadurch gekennzeichnet, dass** sie ein Faktor XΔ-Analogon mit hoher Stabilität und struktureller Integrität des Moleküls enthält.

26. Präparation nach einem der Ansprüche 20 bis 25, **dadurch gekennzeichnet, dass** sie ein Faktor XΔ-Analogon enthält, das eine Modifikation aufweist, die eine Aktivierung von Faktor XΔ-Analogon zu Faktor Xa- Analogon in vitro erlaubt.

27. Präparation nach einem der Ansprüche 20 bis 26, **dadurch gekennzeichnet, dass** sie als pharmazeutisches Präparat formuliert ist.

28. Präparation nach Anspruch 27, **dadurch gekennzeichnet, dass** sie in einer geeigneten Vorrichtung, vorzugsweise einer Applikationsvorrichtung, in Kombination mit einer Protease, ausgewählt aus der Gruppe der Endoproteasen, wie etwa Kexin/Kex2, Furin/PACE, PC1/PC3, PC2, PC4, PACE 4, LPC/PC7, der Serinproteasen, wie etwa Faktor IIa, Faktor VIIa, Faktor Ia, Faktor XIIa, Faktor XIa, Faktor Xa oder von Kallikrein, oder einem Derivat dieser Proteasen, vorliegt.

29. Präparation nach Anspruch 28, **dadurch gekennzeichnet, dass** die Komponenten räumlich voneinander getrennt vorliegen.

30. Präparation enthaltend ein gereinigtes Faktor Xa-Analogon mit hoher Stabilität und struktureller Integrität, das insbesondere frei ist von inaktiven Faktor XΔ/Xa-Analogon-Intermediaten und autoproteolytischen Abbauprodukten, erhältlich durch Aktivierung eines Faktor XΔ-Analogons gemäß einem der Ansprüche 1 bis 17.

31. Präparation nach Anspruch 30, **dadurch gekennzeichnet, dass** sie ein aktives Faktor Xa-Analogon als zweikettiges Molekül in isolierter Form enthält.

32. Präparation nach einem der Ansprüche 30 oder 31, **dadurch gekennzeichnet, dass** sie Faktor Xa-Analogon mit einer Reinheit von mindestens 80%, vorzugsweise 90 %, besonders bevorzugt 95%, enthält und keine inaktiven, proteolytischen Intermediate von Faktor X/Xa-Analogon aufweist.

33. Präparation nach einem der Ansprüche 30 bis 32, **dadurch gekennzeichnet, dass** sie einen physiologisch akzeptablen Träger enthält und in lagerstabiler Form vorliegt.

34. Präparation nach einem der Ansprüche 20 bis 33, **dadurch gekennzeichnet, dass** sie gegebenenfalls als weiteren Bestandteil einen Blutfaktor oder eine aktivierte Form eines Blutfaktors enthält.

35. Präparation nach Anspruch 34, **dadurch gekennzeichnet, dass** sie als weiteren Bestandteil mindestens eine Komponente mit Faktor VIII Bypass-Aktivität enthält.

36. Präparation nach einem der Ansprüche 20 bis 35, **dadurch gekennzeichnet, dass** sie als pharmazeutische Zusammensetzung formuliert ist.

37. Verwendung einer Präparation nach einem der Ansprüche 20 bis 36 zur Herstellung eines Arzneimittels.

38. Verwendung einer Präparation nach einem der Ansprüche 20 bis 36 zur Herstellung eines Arzneimittels zur Behandlung von Patienten mit Störungen der Blutgerinnung, wie etwa Hämophilie-Patienten oder hämophile Patienten, welche Inhibitor-Antikörper entwickelt haben.

39. Verfahren zur Herstellung einer Präparation enthaltend gereinigtes rekombinantes Faktor XΔ-Analogon, **dadurch gekennzeichnet, dass** ein durch rekombinante Herstellung gewonnenes Faktor XΔ-Analogon als einzelkettiges Molekül isoliert und über ein chromatographisches Verfahren gereinigt wird.

40. Verfahren nach Anspruch 39, **dadurch gekennzeichnet, dass** es die folgenden Schritte umfasst:
- Bereitstellen einer Nukleinsäure kodierend für ein Faktor XΔ-Analogon gemäß einem der Ansprüche 1 bis 17
- Transfektion einer geeigneten Zelle
- Expression des Faktor XΔ-Analogons
- Isolieren von einzelkettigem Faktor XΔ-Analogon und
- Reinigung des Polypeptids.

41. Verfahren zur Herstellung einer Präparation enthaltend aktives Faktor XΔ-Analogon, **dadurch gekennzeichnet, dass** eine gemäß einem der Ansprüche 39 oder 40 hergestellte Präparation einem Aktivierungsschritt unterzogen wird.

42. Verfahren nach Anspruch 41, **dadurch gekennzeichnet, dass** die Präparation enthaltend einzelkettiges Faktor XΔ-Analogon mit einer Protease, ausgewählt aus der Gruppe der Endoproteasen, wie etwa Kexin/Kex2, Furin/PACE, PC1/PC3, PC2, PC4, PACE4, LPC/PC7, der Serinproteasen, wie etwa Faktor IIa, Faktor VIIa, Faktor Ixa, Faktor XIIa, Faktor XIa, Faktor Xa oder von Kallikrein, oder einem Derivat dieser Proteasen, in Kontakt gebracht wird, unter Bedingungen, die es erlauben, dass es in die zweikettige Faktor Xa-Analogon-Form gespalten wird.

43. Verfahren nach Anspruch 42, **dadurch gekennzeichnet, dass** die Protease immobilisiert ist.

44. Verfahren nach einem der Ansprüche 40 bis 43, **dadurch gekennzeichnet, dass** ein gereinigtes Faktor Xa-Analogon mit hoher Stabilität und struktureller Integrität, das insbesondere frei ist von inaktiven Faktor XΔ/Xa-Analogon-Intermediaten, gewonnen wird.

## Claims

1. Factor XΔ analogue, **characterised in that** it has a deletion of amino acids Arg180 to Arg234 of the factor XΔ amino acid sequence and a modification in the region of the amino acid sequence between Gly173 and Arg179, wherein said modification represents a processing site of a protease not naturally cleaving at this position of the factor X sequence.

2. Factor XΔ analogue according to claim 1, **characterised in that** said modification is, at least, an amino acid exchange in the region of the amino acid sequence between Glyl73 and Arg179, based on the amino acid numbering as shown in Fig. 1.

3. Factor XΔ analogue according to claim 1 or 2, **characterised in that** it contains a factor X sequence having Glyl73-R6-R5-R4-R3-R2-Arg179/R1(235), wherein
a) R1 is an amino acid selected from the group of Val, Ser, Thr, Ile or Ala,
b) R2 is an amino acid selected from the group of Glu, Thr, Pro, Gly, Lys or Arg,
c) R3 is an amino acid selected from the group of Leu, Phe, Lys, Met, Gln, Glu, Ser, Val, Arg or Pro,
d) R4 is an amino acid selected from the group of Thr, Asp, Asn, Ile, Ser, Met, Pro, Arg or Lys,
e) R5 is an amino acid selected from the group of Asn, Lys, Ser, Glu, Gln, Ala, His or Arg, and
f) R6 is an amino acid selected from the group of Asp, Phe, Thr, Arg, Leu or Ser.

4. Factor XΔ analogue according to any one of claims 1 to 3, **characterised in that** said modification represents a processing site for a protease selected from the group of endoproteases, such as kexin/Kex2, furin/PACE, PC1/PC3, PC2, PC4, PACE 4, LPC/PC7, serine proteases, such as factor IIa, factor VIIa, factor IXa, factor XIIa, factor XIa, factor Xa, or kallikrein, or a derivative of these proteases.

5. Factor XΔ analogue according to any one of claims 1 to 4, **characterised in that** it is present as a single chain molecule in enzymatically inactive form.

6. Factor XΔ analogue according to any one of claims 1 to 5, **characterised in that** said modification allows activation of the inactive, single chain factor XΔ analogue polypeptide to the double chain, active factor Xa analogue form.

7. Factor XΔ analogue according to any one of claims 1 to 6, **characterised in that** it has a further modification in the region of the C-terminal factor X amino acid sequence.

8. Factor XΔ analogue according to claim 7, **characterised in that** it has a modification in the C-terminal region of the β-peptide cleavage site.

9. Factor XΔ analogue according to claim 8, **characterised in that** said modification is a mutation, deletion or insertion in the region of the factor X amino acid sequence between amino acid positions Arg469 and Ser476.

10. Factor XΔ analogue according to any one of claims 7 to 9, **characterised in that** said modification prevents the β-peptide from cleaving off.

11. Factor XΔ analogue according to claim 7, **characterised in that** it has a deletion of the factor Xβ-peptide.

12. Factor XΔ analogue according to claim 11, **characterised in that** it has a translation stop signal in the C-terminal region of the factor X sequence.

13. Factor XΔ analogue according to claim 12, **characterised in that** it has a translation stop signal at the position of amino acid Lys470 of the factor X sequence.

14. Factor XΔ analogue according to any one of claims 1 to 13, **characterised in that** said modification in the region of the amino acid sequence between Gly173 and Arg179 allows activation of the inactive factor X analogue to active factor XΔ analogue in vitro.

15. Factor XΔ analogue according to claim 14, **characterised in that** said modification allows activation by a protease selected from the group of endoproteases, such as kexin/Kex2, furin/PACE, PC1/PC3, PC2, PC4, PACE 4, LPC/PC7, serine proteases, such as factor IIa, factor VIIa, factor IXa, factor XIIa, factor XIa, factor Xa, or kallikrein, or a derivative of these proteases.

16. Factor XΔ analogue according to any one of claims 1 to 15, **characterised in that** it has an intact β-peptide and is provided as factor XΔα.

17. Factor XΔ analogue according to any one of claims 1 to 15, **characterised in that** it has a deletion of the β-peptide.

18. Recombinant DNA coding for a factor XΔ analogue according to any one of claims 1 to 17, contained in a vector for recombinant expression of the encoded protein.

19. Transformed cells containing a recombinant DNA according to claim 18.

20. Preparation containing purified factor XΔ analogue, which has a deletion of amino acids Arg180 to Arg234 of the factor X amino acid sequence and a modification in the region of the amino acid sequence between Gly173 and Arg179, wherein said modification represents a processing site of a protease not naturally cleaving at this position of the factor X sequence.

21. Preparation according to claim 20, **characterised in that** it contains a single chain factor XΔ analogue in enzymatically inactive form having a purity of at least 80%, preferably 90%, particularly preferably 95%, and that it does not contain any inactive, proteolytic intermediates of factor X/Xa analogue.

22. Preparation according to any one of claims 20 or 21, **characterised in that** it contains factor XΔ analogue as factor XΔα.

23. Preparation according to any one of claims 20 or 21, **characterised in that** it contains factor XΔ analogue as FXΔβ.

24. Preparation according to any one of claims 20 to 23, **characterised in that** it contains factor XΔ analogue as a single chain molecule in isolated form.

25. Preparation according to any one of claims 20 to 24, **characterised in that** it contains a factor XΔ analogue having high stability and structural integrity of the molecule.

26. Preparation according to any one of claims 20 to 25, **characterised in that** it contains a factor XΔ analogue having a modification which allows activation of factor XΔ analogue to factor Xa analogue in vitro.

27. Preparation according to any one of claims 20 to 26, **characterised in that** it is formulated as a pharmaceutical preparation.

28. Preparation according to claim 27, **characterised in that** it is present in an appropriate device, preferably an application device, in combination with a protease selected from the group of endoproteases, such as kexin/Kex2, furin/PACE, PC1/PC3, PC2, PC4, PACE 4, LPC/PC7, serine proteases, such as factor IIa, factor VIIa, factor IXa, factor XIIa, factor XIa, factor Xa, or kallikrein, or a derivative of these proteases.

29. Preparation according to claim 28, **characterised in that** the components are provided spatially separated.

30. Preparation containing a purified factor Xa analogue having high stability and structural integrity, which is particularly free of inactive factor XΔ/Xa analogue intermediates and auto-proteolytic degradation products, obtainable by activating a factor XΔ analogue according to any one of claims 1 to 17.

31. Preparation according to claim 30, **characterised in that** it contains an active factor Xa analogue as a double chain molecule in isolated form.

32. Preparation according to any one of claims 30 or 31, **characterised in that** it contains factor Xa analogue having a purity of at least 80%, preferably 90%, particularly preferably 95%, and does not contain any inactive, proteolytic intermediates of factor X/Xa analogue.

33. Preparation according to any one of claims 30 to 32, **characterised in that** it contains a physiologically acceptable carrier and is provided in stably storable form.

34. Preparation according to any one of claims 20 to 33, **characterised in that** it optionally contains a blood factor or an activated form of a blood factor as a further component.

35. Preparation according to claim 34, **characterised in that** it contains at least one component having factor VIII bypass activity as a further component.

36. Preparation according to any one of claims 20 to 35, **characterised in that** it is formulated as a pharmaceutical composition.

37. Use of a preparation according to any one of claims 20 to 36 for preparing a medicament.

38. Use of a preparation according to any one of claims 20 to 36 for preparing a medicament for the treatment of patients suffering from blood coagulation disorders, such as patients suffering from hemophilia or hemophiliacs who have developed inhibitor antibodies.

39. Process for preparing a preparation containing purified recombinant factor XΔ analogue, **characterised in that** a factor XΔ analogue obtained by recombinant preparation is isolated as a single chain molecule and purified by means of a chromatographic process.

40. Process according to claim 39, **characterised in that** it comprises the following steps:
- providing a nucleic acid encoding a factor XΔ analogue according to any one of claims 1 to 17
- transfection of an appropriate cell
- expression of the factor XΔ analogue
- isolation of the single chain factor XΔ analogue, and
- purification of the polypeptide.

41. Process for preparing a preparation containing active factor XΔ analogue, **characterised in that** a preparation prepared according to any one of claims 39 or 40 is subjected to an activation step.

42. Process according to claim 41, **characterised in that** the preparation containing single chain factor XΔ analogue is brought into contact with a protease selected from the group of endoproteases, such as kexin/Kex2, furin/PACE, PC1/PC3, PC2, PC4, PACE 4, LPC/PC7, serine proteases, such as factor IIa, factor VIIa, factor IXa, factor XIIa, factor XIa, factor Xa, or kallikrein, or a derivative of these proteases, under conditions allowing cleavage to the double chain factor Xa analogue form.

43. Process according to claim 42, **characterised in that** the protease is immobilized.

44. Process according to any one of claims 40 to 43, **characterised in that** a purified factor Xa analogue having high stability and structural integrity is obtained, which is particularly free of inactive factor XΔ/Xa analogue intermediates.

## Revendications

1. Analogue du facteur XΔ, **caractérisé en ce qu'**il présente une délétion des acides aminés Arg180 à Arg234 de la séquence d'acides aminés du facteur XΔ et une modification dans le domaine de la séquence d'acides aminés entre Gly173 et Arg179, dans lequel la modification correspond à un site de maturation d'une protéase clivée de façon non naturelle à cet endroit de la séquence du facteur X.

2. Analogue du facteur XΔ selon la revendication 1, **caractérisé en ce que** la modification est au moins un échange d'acides aminés dans le domaine de la séquence d'acides aminés entre Gly173 et Arg179 conformément à la numérotation des acides aminés selon la figure 1.

3. Analogue du facteur XΔ selon la revendication 1 ou 2, **caractérisé en ce qu'**il contient une séquence du facteur X avec Gly173-R6-R5-R4-R3-R2-Arg179/R1(235), dans laquelle
a) R1 est un acide aminé choisi dans le groupe constitué de Val, Ser, Thr, Ile ou Ala
b) R2 est un acide aminé choisi dans le groupe constitué de Glu, Thr, Pro, Gly, Lys ou Arg
c) R3 est un acide aminé choisi dans le groupe constitué de Leu, Phe, Lys, Met, Gln, Glu, Ser, Val, Arg ou Pro
d) R4 est un acide aminé choisi dans le groupe constitué de Thr, Asp, Asn, Ile, Ser, Met, Pro, Arg ou Lys
e) R5 est un acide aminé choisi dans le groupe constitué de Asn, Lys, Ser, Glu, Gln, Ala, His ou Arg et
f) R6 est un acide aminé choisi dans le groupe constitué de Asp, Phe, Thr, Arg, Leu ou Ser.

4. Analogue du facteur XΔ selon l'une des revendications 1 à 3, **caractérisé en ce que** la modification correspond à un site de maturation d'une protéase choisie dans le groupe des endoprotéases, telles que la kexine/kex2, la furine/PACE, PC1/PC3, PC2, PC4, PACE 4, LPC/PC7, les protéases sérines, telles que le facteur IIa, le facteur VIIa, le facteur IXa, le facteur XIIa, le facteur XIa, le facteur Xa, ou la kallicréine, ou un dérivé de ces protéases.

5. Analogue du facteur XΔ selon l'une quelconque des revendications 1 à 4, **caractérisé en ce qu'**il se présente comme une molécule à chaîne simple sous une forme enzymatiquement inactivée.

6. Analogue du facteur XΔ selon l'une des revendications 1 à 5, **caractérisé en ce que** la modification permet une activation du polypeptide de l'analogue du facteur XΔ à chaîne simple inactivé sous la forme d'analogue du facteur Xa actif à double chaîne.

7. Analogue du facteur XΔ selon l'une des revendications 1 à 6, **caractérisé en ce qu'**il présente une autre modification dans le domaine de la séquence d'acides aminés C-terminale du facteur X.

8. Analogue du facteur XΔ selon la revendication 7, **caractérisé en ce qu'**il présente une modification dans le domaine C-terminal du site de clivage du peptide β.

9. Analogue du facteur XΔ selon la revendication 8, **caractérisé en ce que** la modification est une mutation, une délétion ou une insertion dans le domaine de la séquence d'acides aminés du facteur X entre les positions d'acides aminés Arg469 et Ser476.

10. Analogue du facteur XΔ selon l'une des revendications 7 à 9, **caractérisé en ce que** la modification empêche le clivage du peptide β.

11. Analogue du facteur XΔ selon la revendication 7, **caractérisé en ce qu'**il présente une délétion du peptide β du facteur X.

12. Analogue du facteur XΔ selon la revendication 11, **caractérisé en ce qu'**il présente un signal d'arrêt de la traduction dans le domaine C-terminal de la séquence du facteur X.

13. Analogue du facteur XΔ selon la revendication 12, **caractérisé en ce qu'**il présente un signal d'arrêt de traduction à la position de l'acide aminé Lys470 de la séquence du facteur X.

14. Analogue du facteur XΔ selon l'une des revendications 1 à 13, **caractérisé en ce que** la modification dans le domaine de la séquence des acides aminés entre Gly173 et Arg179 permet une activation in vitro de l'analogue du facteur X inactivé en un analogue du facteur XΔ activé.

15. Analogue du facteur XΔ selon la revendication 14, **caractérisé en ce que** la modification permet une activation grâce à une protéase, choisie dans le groupe des endoprotéases, telles que la kexine/kex2, la furine/PACE, PC1/PC3, PC2, PC4, PACE 4, LPC/PC7, les protéases sérines, telles que le facteur IIa, le facteur VIIa, le facteur IXa, le facteur XIIa, le facteur XIa, le facteur Xa, ou la kallicréine, ou un dérivé de ces protéases.

16. Analogue du facteur XΔ selon l'une des revendications 1 à 15, **caractérisé en ce qu'**il présente un peptide β intact et qu'il se présente sous la forme du facteur XΔα.

17. Analogue du facteur XΔ selon l'une des revendications 1 à 15, **caractérisé en ce qu'**il présente une délétion du peptide β.

18. ADN recombiné, codant pour un analogue du facteur XΔ selon l'une des revendications 1 à 17, contenant un vecteur pour l'expression recombinée de la protéine codée.

19. Cellules transformées contenant un ADN recombiné selon la revendication 18.

20. Préparation contenant l'analogue du facteur XΔ purifié, qui présente une délétion des acides aminés Arg180 à ARG234 de la séquence d'acides aminés du facteur X et une modification dans le domaine de la séquence d'acides aminés entre Gly173 et Arg179, dans laquelle la modification correspond à un site de maturation d'une protéase clivée non naturellement à cet endroit de la séquence du facteur X.

21. Préparation selon la revendication 20, **caractérisée en ce qu'**elle contient un analogue du facteur XΔ à chaîne simple sous une forme enzymatiquement inactivée d'une pureté d'au moins 80%, de préférence 90%, de façon particulièrement préférée 95%, et ne présente aucun intermédiaire protéolytique inactivé de l'analogue du facteur X/Xa.

22. Préparation selon la revendication 20 ou 21, **caractérisée en ce qu'**elle contient l'analogue du facteur XΔ comme facteur XΔα.

23. Préparation selon la revendication 20 ou 21, **caractérisée en ce qu'**elle contient l'analogue du facteur XΔ comme FXΔβ.

24. Préparation selon la revendication 20 à 23, **caractérisée en ce qu'**elle contient l'analogue du facteur XΔ comme molécule à chaîne simple sous une forme isolée.

25. Préparation selon l'une des revendications 20 à 24, **caractérisée en ce qu'**elle contient un analogue du facteur XΔ d'une grande stabilité et intégrité structurelle de la molécule.

26. Préparation selon l'une des revendications 20 à 25, **caractérisée en ce qu'**elle contient un analogue du facteur XΔ, qui présente une modification, qui permet une activation *in vitro* de l'analogue du facteur XΔ en analogue du facteur Xa.

27. Préparation selon l'une des revendications 20 à 26, **caractérisée en ce qu'**elle est formulée en tant que préparation pharmaceutique.

28. Préparation selon la revendication 27, **caractérisée en ce qu'**elle se présente dans un dispositif approprié, de préférence un dispositif d'application, en combinaison avec une protéase, choisie dans le groupe des endoprotéases, telles que la kexine/kex2, la furine/PACE, PC1/PC3, PC2, PC4, PACE 4, LPC/PC7, les protéases sérines, telles que le facteur IIa, le facteur VIIa, le facteur IXa, le facteur XIIa, le facteur XIa, le facteur Xa, ou la kallicréine, ou un dérivé de ces protéases.

29. Préparation selon la revendication 28, **caractérisée en ce que** les composants se présentent de façon séparée au plan spatial.

30. Préparation contenant un analogue du facteur XΔ purifié avec une grande stabilité et intégrité structurelle qui est particulièrement vierge de tout intermédiaire d'analogue du facteur XΔ/Xa inactivé et de produits de dégradation autoprotéolytique, que l'on peut obtenir par activation d'un analogue du facteur XΔ selon l'une des revendications 1 à 17.

31. Préparation selon la revendication 30, **caractérisée en ce qu'**elle contient un analogue du facteur XΔ activé comme molécule double sous forme isolée.

32. Préparation selon l'une des revendications 30 ou 31, **caractérisée en ce qu'**elle contient l'analogue du facteur Xa avec une pureté d'au moins 80%, de préférence 90%, de façon particulièrement préférée 95%, et ne présente pas d'intermédiaires protéolytiques inactivés de l'analogue du facteur X/Xa.

33. Préparation selon l'une des revendications 30 ou 32, **caractérisée en ce qu'**elle contient un véhicule physiologiquement acceptable et se présente sous une forme stable en vue du stockage.

34. Préparation selon l'une des revendications 20 ou 33, **caractérisée en ce qu'**elle contient éventuellement un facteur sanguin ou une forme activée d'un facteur sanguin comme autre composant.

35. Préparation selon la revendication 34, **caractérisée en ce qu'**elle contient au moins un composant ayant l'activité de contournement du facteur VIII comme autre composant.

36. Préparation selon l'une des revendications 20 à 35, **caractérisée en ce qu'**elle est formulée comme composition pharmaceutique.

37. Utilisation d'une préparation selon l'une des revendications 20 à 36 pour la fabrication d'un médicament.

38. Utilisation d'une préparation selon l'une des revendications 20 à 36 pour la fabrication d'un médicament pour le traitement de patient ayant des troubles de la coagulation, comme des patients avec par exemple une hémophilie ou hémophiles, qui ont développé des anticorps inhibiteurs.

39. Procédé de fabrication d'une préparation contenant l'analogue du facteur X△ recombiné purifié, **caractérisé en ce qu'**un analogue du facteur XΔ obtenu par fabrication recombinée est isolé sous la forme d'une molécule à chaîne simple et purifié grâce à un procédé chromatographique.

40. Procédé selon la revendication 39, **caractérisé en ce qu'**il comprend les étapes suivantes :
- préparation d'un acide nucléique codant pour un analogue du facteur XΔ selon l'une des revendications 1 à 17
- transfection d'une cellule appropriée
- expression de l'analogue du facteur X△
- isolement de l'analogue à chaîne simple du facteur XΔ
- purification du polypeptide.

41. Procédé de fabrication d'une préparation contenant l'analogue du facteur XΔ activé, **caractérisé en ce que** la préparation fabriquée selon l'une des revendications 39 ou 40 est soumise à une étape d'activation.

42. Procédé selon la revendication 41, **caractérisé en ce que** la préparation contenant l'analogue à chaîne simple du facteur XΔ est mise en contact avec une protéase choisie dans le groupe des endoprotéases, telles que la kexine/kex2, la furine/PACE, PC1/PC3, PC2, PC4, PACE 4, LPC/PC7, les protéases sérines, telles que le facteur IIa, le facteur VIIa, le facteur IXa, le facteur XIIa, le facteur XIa, le facteur Xa, ou la kallicréine, ou un dérivé de ces protéases, dans des conditions qui lui permettent d'être clivée en une forme à chaîne double de l'analogue du facteur Xa.

43. Procédé selon la revendication 42, **caractérisé en ce que** la protéase est immobilisée.

44. Procédé selon l'une des revendications 40 à 43, **caractérisé en ce qu'**on obtient un analogue du facteur Xa purifié avec une grande stabilité et intégrité structurelle, qui est particulièrement vierge de tout intermédiaire de l'analogue du facteur XΔ/Xa inactivé.


### SEQUENZPROTOKOLL

(1) ALLGEMEINE ANGABEN:
   (i) ANMELDER:
      (A) NAME: IMMUNO AG
      (B) STRASSE: Industriestrasse 67
      (C) ORT: Wien
      (D) BUNDESLAND: Austria
      (E) LAND: Austria
      (F) POSTLEITZAHL: 1220

      (A) NAME: Friedrich Dorner
      (B) STRASSE: Peterlinigasse 17
      (C) ORT: Wien
      (D) BUNDESLAND: Austria
      (E) LAND: Austria
      (F) POSTLEITZAHL: 1238

      (A) NAME: Falko-Guenther Falkner
      (B) STRASSE: Mannsdorf 116
      (C) ORT: Mannsdorf
      (D) BUNDESLAND: Austria
      (E) LAND: Austria
      (F) POSTLEITZAHL: 2304

      (A) NAME: Michele Himmelspach
      (B) STRASSE: Breitstetten 19
      (C) ORT: Leopoldsdorf
      (D) BUNDESLAND: Austria
      (E) LAND: Austria
      (F) POSTLEITZAHL: 2285

      (A) NAME: Michael Pfleiderer
      (B) STRASSE: Johann Nestroygasse 12/16
      (C) ORT: Gross-Enzersdorf
      (D) BUNDESLAND: Austria
      (E) LAND: Austria
      (F) POSTLEITZAHL: 2301

      (A) NAME: Uwe Schlokat
      (B) STRASSE: Haupstrasse 51
      (C) ORT: Orth/Donau
      (D) BUNDESLAND: Austria
      (E) LAND: Austria
      (F) POSTLEITZAHL: 2304

      (A) NAME: Johann Eibl
      (B) STRASSE: Gustav Tschermakgasse 2
      (C) ORT: Wien
      (D) BUNDESLAND: Austria
      (E) LAND: Austria
      (F) POSTLEITZAHL: 1180
   (ii) BEZEICHNUNG DER ERFINDUNG: Faktor X-Deltionsmutanten und Analoge davon
   (iii) ANZAHL DER SEQUENZEN: 44
   (iv) COMPUTER-LESBARE FASSUNG:
      (A) DATENTRÄGER : Floppy disk
      (B) COMPUTER: IBM PC compatible
      (C) BETRIEBSSYSTEM : PC-DOS/MS-DOS
      (D) SOFTWARE: Patent In Release #1.0, Version #1.30 (EPA)
(2) ANGABEN ZU SEQ ID NO: 1 :
   (i) SEQUENZKENNZEICHEN :
      (A) LÄNGE: 34 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 1:
      ATTACTCGAG AAGCTTACCA TGGGGCGCCC ACTG 34
(2) ANGABEN ZU SEQ ID NO: 2:
   (i) SEQUENZKENNZEICHEN:
      (A) LÄNGE: 24 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 2:
      ATTACAATTG CTGCAGGGAT CCAC 24
(2) ANGABEN ZU SEQ ID NO: 3:
   (i) SEQUENZKENNZEICHEN:
      (A) LÄNGE: 38 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 3:
      GGCCCTACCC CTGTGGGAAA CAGGACTTCA CCAGGGTG 38
(2) ANGABEN ZU SEQ ID NO: 4:
   (i) SEQUENZKENNZEICHEN:
      (A) LÄNGE: 34 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 4:
      CACCCTGGTG AAGTCCTGTT TCCCACAGGG GTAG 34
(2) ANGABEN ZU SEQ ID NO: 5:
   (i) SEQUENZKENNZEICHEN:
      (A) LÄNGE: 38 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 5:
      GGCCCTACCC CTGTGGGAAA CAGACCCTGG AACGGACC 38
(2) ANGABEN ZU SEQ ID NO: 6:
   (i) SEQUENZKENNZEICHEN:
      (A) LÄNGE: 34 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 6:
      GGTCCGTTCC AGGGTCTGTT TCCCACAGGG GTAG 34
(2) ANGABEN ZU SEQ ID NO: 7:
   (i) SEQUENZKENNZEICHEN:
      (A) LÄNGE: 38 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 7:
      GGCCCTACCC CTGTGGGAAA CAGATCAAGC CCAGGATC 38
(2) ANGABEN ZU SEQ ID NO: 8:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 8:
      CTGGGCTTGA TCTGTTTCCC ACAGGGGTAG 30
(2) ANGABEN ZU SEQ ID NO: 9:
   (i) SEQUENZKENNZEICHEN:
      (A) LÄNGE: 38 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 9:
      GGCCCTACCC CTGTGGGAAA CAGAGCATGA CCAGGATC 38
(2) ANGABEN ZU SEQ ID NO: 10:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 10:
      CTGGTCATGC TCTGTTTCCC ACAGGGGTAG 30
(2) ANGABEN ZU SEQ ID NO: 11 :
   (i) SEQUENZKENNZEICHEN:
      (A) LÄNGE: 38 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 11:
      GGCCCTACCC CTGTGGGAAA CAGATGAAAA CGAGGATC 38
(2) ANGABEN ZU SEQ ID NO: 12:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG : SEQ ID NO: 12:
      CTCGTTTTCA TCTGTTTCCC ACAGGGGTAG 30
(2) ANGABEN ZU SEQ ID NO: 13:
   (i) SEQUENZKENNZEICHEN:
      (A) LÄNGE: 38 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 13:
      GGCCCTACCC CTGTGGGAAA CAGATCGAGG GAAGGATC 38
(2) ANGABEN ZU SEQ ID NO: 14:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS : Genom-DNA
   (xi) SEQUENZBESCHREIBUNG: SEQ ID NO: 14:
      CTTCCCTCGA TCTGTTTCCC ACAGGGGTAG 30
(2) ANGABEN ZU SEQ ID NO: 15:
   (i) SEQUENZKENNZEICHEN:
      (A) LÄNGE: 38 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 15:
      GGCCCTACCC CTGTGGGAAA CAGAGGAGGA AGAGGATC 38
(2) ANGABEN ZU SEQ ID NO: 16:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 16:
      CTCTTCCTCC TCTGTTTCCC ACAGGGGTAG 30
(2) ANGABEN ZU SEQ ID NO: 17:
   (i) SEQUENZKENNZEICHEN:
      (A) LÄNGE: 38 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 17:
      GGCCCTACCC CTGTGGGAAA CAGAGGGTGA GGAGGATC 38
(2) ANGABEN ZU SEQ ID NO: 18:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 18:
      CTCCTCACCC TCTGTTTCCC ACAGGGGTAG 30
(2) ANGABEN ZU SEQ ID NO: 19:
   (i) SEQUENZKENNZEICHEN:
      (A) LÄNGE: 38 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 19:
      GGCCCTACCC CTGTGGGAAA CAGAGGAGGA GGAGGATC 38
(2) ANGABEN ZU SEQ ID NO: 20:
   (i) SEQUENZKENNZEICHEN :
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 20:
      CTCCTCCTCC TCTGTTTCCC ACAGGGGTAG 30
(2) ANGABEN ZU SEQ ID NO: 21:
   (i) SEQUENZKENNZEICHEN:
      (A) LÄNGE: 38 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 21:
      GGCCCTACCC CTGTGGGAAA CAGAGGCCCA AGAGGATC 38
(2) ANGABEN ZU SEQ ID NO: 22:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 22:
      CTCTTGGGCC TCTGTTTCCC ACAGGGGTAG 30
(2) ANGABEN ZU SEQ ID NO: 23:
   (i) SEQUENZKENNZEICHEN:
      (A) LÄNGE: 38 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 23:
      GGCCCTACCC CTGTGGGAAA CAGATCAGGA AGAGGATC 38
(2) ANGABEN ZU SEQ ID NO: 24:
   (i) SEQUENZKENNZEICHEN:
      (A) LÄNGE: 30 Basenpaare
      (B) ART: Nucleotid
      (C) STRANGFORM: Einzelscrang
      (D) TOPOLOGIE: linear
   (ii) ART DES MOLEKÜLS: Genom-DNA
   (xi) SEQUENZBESCHREIBUNG: SEQ ID NO: 24:
      CTCTTCGTGA TCTGTTTCCC ACAGGGGTAG 30
(2) ANGABEN ZU SEQ ID NO: 25:
   (i) SEQUENZKENNZEICHEN:
      (A) LÄNGE: 38 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 25:
      GGCCCTACCC CTGTGGGAAA CAGAGGAGCA AGAGGATC 38
(2) ANGABEN ZU SEQ ID NO: 26:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 26:
      CTCTTGCTCC TCTGTTTCCC ACAGGGGTAG 30
(2) ANGABEN ZU SEQ ID NO: 27:
   (i) SEQUENZKENNZEICHEN:
      (A) LÄNGE: 38 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 27:
      GGCCCTACCC CTGTGGGAAA CAGAGGGTCA CGAGGATC 38
(2) ANGABEN ZU SEQ ID NO: 28:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 28:
      CTCGTGACCC TCTGTTTCCC ACAGGGGTAG 30
(2) ANGABEN ZU SEQ ID NO: 29:
   (i) SEQUENZKENNZEICHEN:
      (A) LÄNGE: 38 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 29:
      GGCCCTACCC CTGTGGGAAA CAGAGGCTGA AAAGGATC 38
(2) ANGABEN ZU SEQ ID NO: 30:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 30:
      CTTTTCAGCC TCTGTTTCCC ACAGGGGTAG 30
(2) ANGABEN ZU SEQ ID NO: 31:
   (i) SEQUENZKENNZEICHEN
      (A) LÄNGE: 38 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 31:
      GGCCCTACCC CTGTGGGAAA CAGCCCCAAG GAAGGATC 38
(2) ANGABEN ZU SEQ ID NO: 32:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 32:
      CTTCCTTGGG GCTGTTTCCC ACAGGGGTAG 30
(2) ANGABEN ZU SEQ ID NO: 33:
   (i) SEQUENZKENNZEICHEN:
      (A) LÄNGE: 38 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 33:
      GGCCCTACCC CTGTGGGAAA CAGACGAGCA CGAGGATC 38
(2) ANGABEN ZU SEQ ID NO: 34:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 34:
      CTCGTGCTCG TCTGTTTCCC ACAGGGGTAG 30
(2) ANGABEN ZU SEQ ID NO: 35:
   (i) SEQUENZKENNZEICHEN:
      (A) LANGE: 38 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 35:
      GGCCCTACCC CTGTGGGAAA CAGACCCTGG AACGGATC 38
(2) ANGABEN ZU SEQ ID NO: 36:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 36:
      CGTTCCAGGG TCTGTTTCCC ACAGGGGTAG 30
(2) ANGABEN ZU SEQ ID NO: 37:
   (i) SEQUENZKENNZEICHEN:
      (A) LÄNGE: 49 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 37:
      GTCACCGCCT TCCTCAAGTG GATCGACAGG TCCATGAAAA CCAGGTGAA 49
(2) ANGABEN ZU SEQ ID NO: 38:
   (i) SEQUENZKENNZEICHEN:
      (A) LÄNGE: 48 Basenpaare
      (B) ART: Nucleotid
      (C) STRANGFORM: Einzelscrang
      (D) TOPOLOGIE: linear
   (ii) ART DES MOLEKÜLS: Genom-DNA
   (xi) SEQUENZBESCHREIBUNG: SEQ ID NO: 38:
      CTAGTTCACC TGGTTTTCAT GGACCTGTCG ATCCACTTGA GGAAGGCG 48
(2) ANGABEN ZU SEQ ID NO: 39:
   (i) SEQUENZKENNZEICHEN:
      (A) LÄNGE: 57 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 39:
      GTCACCGCCT TCCTCAAGTG GATCGACAGG TCCATGAAAA CCAAGGGCTT GCCCAAG 57
(2) ANGABEN ZU SEQ ID NO: 40:
   (i) SEQUENZKENNZEICHEN:
      (A) LÄNGE: 57 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 40:
      TTGGCCTTGG GCAAGCCCTT GGTTTTCATG GACCTGTCGA TCCACTTGAG GAAGGCG 57
(2) ANGABEN ZU SEQ ID NO: 41:
   (i) SEQUENZKENNZEICHEN:
      (A) LÄNGE: 55 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 41:
      GCCAAGAGCC ATGCCCCGGA GGTCATAACG TCCTCTCCAT TAAAGTGAGA TCCCA 55
(2) ANGABEN ZU SEO ID NO: 42:
   (i) SEQUENZKENNZEICHEN
      (A) LÄNGE: 54 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG : SEQ ID NO: 42:
      CTAGTGGGAT CTCACTTTAA TGGAGAGGAC GTTATGACCT CCGGGGCATG GCTC 54
(2) ANGABEN ZU SEQ ID NO: 43:
   (i) SEQUENZKENNZEICHEN :
      (A) LÄNGE: 1467 Basenpaare
      (B) ART: Nucleotid
      (C) STRANGFORM: Einzelstrang
      (D) TOPOLOGIE: linear
   (ii) ART DES MOLEKÜLS: Genom-DNA
   (ix) MERKMAL:
      (A) NAME/SCHLÜSSEL: CDS
      (B) LAGE:1..1467
   (xi) SEQUENZBESCHREIBUNG: SEQ ID NO: 43:
   ATG GGG CGC CCA CTG CAC CTC GTC CTG CTC AGT GCC TCC CTG GCT GGC      48
   Met Gly Arg Pro Leu His Leu Val Leu Leu Ser Ala Ser Leu Ala Gly
    1               5                  10                  15
   CTC CTG CTG CTC GGG GAA AGT CTG TTC ATC CGC AGG GAG CAG GCC AAC      96
   Leu Leu Leu Leu Gly Glu Ser Leu Phe Ile Arg Arg Glu Gln Ala Asn
               20                  25                  30
   AAC ATC CTG GCG AGG GTC ACG AGG GCC AAT TCC TTT CTT GAA GAG ATG     144
   Asn Ile Leu Ala Arg Val Thr Arg Ala Asn Ser Phe Leu Glu Glu Met
           35                  40                  45
   AAG AAA GGA CAC CTC GAA AGA GAG TGC ATG GAA GAG ACC TGC TCA TAC     192
   Lys Lys Gly His Leu Glu Arg Glu Cys Met Glu Glu Thr Cys Ser Tyr
       50                  55                  60
   GAA GAG GCC CGC GAG GTC TTT GAG GAC AGC GAC AAG ACG AAT GAA TTC     240
   Glu Glu Ala Arg Glu Val Phe Glu Asp Ser Asp Lys Thr Asn Glu Phe
   65                  70                  75                  80
   TGG AAT AAA TAC AAA GAT GGC GAC CAG TGT GAG ACC AGT CCT TGC CAG     288
   Trp Asn Lys Tyr Lys Asp Gly Asp Gln Cys Glu Thr Ser Pro Cys Gln
                   85                  90                  95
   AAC CAG GGC AAA TGT AAA GAC GGC CTC GGG GAA TAC ACC TGC ACC TGT     336
   Asn Gln Gly Lys Cys Lys Asp Gly Leu Gly Glu Tyr Thr Cys Thr Cys
               100                 105                 110
   TTA GAA GGA TTC GAA GGC AAA AAC TGT GAA TTA TTC ACA CGG AAG CTC     384
   Leu Glu Gly Phe Glu Gly Lys Asn Cys Glu Leu Phe Thr Arg Lys Leu
           115                 120                 125
   TGC AGC CTG GAC AAC GGG GAC TGT GAC CAG TTC TGC CAC GAG GAA CAG     432
   Cys Ser Leu Asp Asn Gly Asp Cys Asp Gln Phe Cys His Glu Glu Gln
       130                 135                 140
   AAC TCT GTG GTG TGC TCC TGC GCC CGC GGG TAC ACC CTG GCT GAC AAC      480
   Asn Ser Val Val Cys Ser Cys Ala Arg Gly Tyr Thr Leu Ala Asp Asn
   145                 150                 155                 160
   GGC AAG GCC TGC ATT CCC ACA GGG CCC TAC CCC TGT GGG AAA CAG ACC      528
   Gly Lys Ala Cys Ile Pro Thr Gly Pro Tyr Pro Cys Gly Lys Gln Thr
                   165                 170                 175
   CTG GAA CGC AGG AAG AGG TCA GTG GCC CAG GCC ACC AGC AGC AGC GGG      576
   Leu Glu Arg Arg Lys Arg Ser Val Ala Gln Ala Thr Ser Ser Ser Gly
               180                 185                 190
   GAG GCC CCT GAC AGC ATC ACA TGG AAG CCA TAT GAT GCA GCC GAC CTG      624
   Glu Ala Pro Asp Ser Ile Thr Trp Lys Pro Tyr Asp Ala Ala Asp Leu
           195                 200                 205
   GAC CCC ACC GAG AAC CCC TTC GAC CTG CTT GAC TTC AAC CAG ACG CAG      672
   Asp Pro Thr Glu Asn Pro Phe Asp Leu Leu Asp Phe Asn Gln Thr Gln
       210                 215                 220
   CCT GAG AGG GGC GAC AAC AAC CTC ACC AGG ATC GTG GGA GGC CAG GAA      720
   Pro Glu Arg Gly Asp Asn Asn Leu Thr Arg Ile Val Gly Gly Gln Glu
   225                 230                 235                 240
   TGC AAG GAC GGG GAG TGT CCC TGG CAG GCC CTG CTC ATC AAT GAG GAA      768
   Cys Lys Asp Gly Glu Cys Pro Trp Gln Ala Leu Leu Ile Asn Glu Glu
                   245                 250                 255
   AAC GAG GGT TTC TGT GGT GGA ACT ATT CTG AGC GAG TTC TAC ATC CTA      816
   Asn Glu Gly Phe Cys Gly Gly Thr Ile Leu Ser Glu Phe Tyr Ile Leu
               260                 265                 270
   ACG GCA GCC CAC TGT CTC TAC CAA GCC AAG AGA TTC AAG GTG AGG GTA      864
   Thr Ala Ala His Cys Leu Tyr Gln Ala Lys Arg Phe Lys Val Arg Val
           275                 280                 285
   GGG GAC CGG AAC ACG GAG CAG GAG GAG GGC GGT GAG GCG GTG CAC GAG      912
   Gly Asp Arg Asn Thr Glu Gln Glu Glu Gly Gly Glu Ala Val His Glu
       290                 295                 300
   GTG GAG GTG GTC ATC AAG CAC AAC CGG TTC ACA AAG GAG ACC TAT GAC      960
   Val Glu Val Val Ile Lys His Asn Arg Phe Thr Lys Glu Thr Tyr Asp
   305                 310                 315                 320
   TTC GAC ATC GCC GTG CTC CGG CTC AAG ACC CCC ATC ACC TTC CGC ATG     1008
   Phe Asp Ile Ala Val Leu Arg Leu Lys Thr Pro Ile Thr Phe Arg Met
                   325                 330                 335
   AAC GTG GCG CCT GCC TGC CTC CCC GAG CGT GAC TGG GCC GAG TCC ACG     1056
   Asn Val Ala Pro Ala Cys Leu Pro Glu Arg Asp Trp Ala Glu Ser Thr
               340                 345                 350
   CTG ATG ACG CAG AAG ACG GGG ATT GTG AGC GGC TTC GGG CGC ACC CAC     1104
   Leu Met Thr Gln Lys Thr Gly Ile Val Ser Gly Phe Gly Arg Thr His
           355                 360                 365
   GAG AAG GGC CGG CAG TCC ACC AGG CTC AAG ATG CTG GAG GTG CCC TAC     1152
   Glu Lys Gly Arg Gln Ser Thr Arg Leu Lys Met Leu Glu Val Pro Tyr
       370                 375                 380
   GTG GAC CGC AAC AGC TGC AAG CTG TCC AGC AGC TTC ATC ATC ACC CAG     1200
   Val Asp Arg Asn Ser Cys Lys Leu Ser Ser Ser Phe Ile Ile Thr Gln
   385                 390                 395                 400
   AAC ATG TTC TGT GCC GGC TAC GAC ACC AAG CAG GAG GAT GCC TGC CAG     1248
   Asn Met Phe Cys Ala Gly Tyr Asp Thr Lys Gln Glu Asp Ala Cys Gln
                   405                 410                 415
   GGG GAC AGC GGG GGC CCG CAC GTC ACC CGC TTC AAG GAC ACC TAC TTC     1296
   Gly Asp Ser Gly Gly Pro His Val Thr Arg Phe Lys Asp Thr Tyr Phe
               420                 425                 430
   GTG ACA GGC ATC GTC AGC TGG GGA GAG AGC TGT GCC CGT AAG GGG AAG     1344
   Val Thr Gly Ile Val Ser Trp Gly Glu Ser Cys Ala Arg Lys Gly Lys
           435                 440                 445
   TAC GGG ATC TAC ACC AAG GTC ACC GCC TTC CTC AAG TGG ATC GAC AGG     1392
   Tyr Gly Ile Tyr Thr Lys Val Thr Ala Phe Leu Lys Trp Ile Asp Arg
       450                 455                 460
   TCC ATG AAA ACC AGG GGC TTG CCC AAG GCC AAG AGC CAT GCC CCG GAG     1440
   Ser Met Lys Thr Arg Gly Leu Pro Lys Ala Lys Ser His Ala Pro Glu
   465                 470                 475                 480
   GTC ATA ACG TCC TCT CCA TTA AAG TGA                                 1467
   Val Ile Thr Ser Ser Pro Leu Lys  *
                   485
(2) ANGABEN ZU SEQ ID NO: 44:
   (i) SEQUENZKENNZEICHEN:
      (A) LÄNGE: 489 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 44:
   Met Gly Arg Pro Leu His Leu Val Leu Leu Ser Ala Ser Leu Ala Gly
    1               5                  10                  15
   Leu Leu Leu Leu Gly Glu Ser Leu Phe Ile Arg Arg Glu Gln Ala Asn
               20                  25                  30
   Asn Ile Leu Ala Arg Val Thr Arg Ala Asn Ser Phe Leu Glu Glu Met
           35                  40                  45
   Lys Lys Gly His Leu Glu Arg Glu Cys Met Glu Glu Thr Cys Ser Tyr
       50                  55                  60
   Glu Glu Ala Arg Glu Val Phe Glu Asp Ser Asp Lys Thr Asn Glu Phe
   65                  70                  75                  80
   Trp Asn Lys Tyr Lys Asp Gly Asp Gln Cys Glu Thr Ser Pro Cys Gln
                   85                  90                  95
   Asn Gln Gly Lys Cys Lys Asp Gly Leu Gly Glu Tyr Thr Cys Thr Cys
   100 105 110
   Leu Glu Gly Phe Glu Gly Lys Asn Cys Glu Leu Phe Thr Arg Lys Leu
   115 120 125
   Cys Ser Leu Asp Asn Gly Asp Cys Asp Gln Phe Cys His Glu Glu Gln
   130 135 140
   Asn Ser Val Val Cys Ser Cys Ala Arg Gly Tyr Thr Leu Ala Asp Asn
   145 150 155 160
   Gly Lys Ala Cys Ile Pro Thr Gly Pro Tyr Pro Cys Gly Lys Gln Thr
   165 170 175
   Leu Glu Arg Arg Lys Arg Ser Val Ala Gln Ala Thr Ser Ser Ser Gly
   180 185 190
   Glu Ala Pro Asp Ser Ile Thr Trp Lys Pro Tyr Asp Ala Ala Asp Leu
   195 200 205
   Asp Pro Thr Glu Asn Pro Phe Asp Leu Leu Asp Phe Asn Gln Thr Gln
   210 215 220
   Pro Glu Arg Gly Asp Asn Asn Leu Thr Arg Ile Val Gly Gly Gln Glu
   225 230 235 240
   Cys Lys Asp Gly Glu Cys Pro Trp Gln Ala Leu Leu Ile Asn Glu Glu
   245 250 255
   Asn Glu Gly Phe Cys Gly Gly Thr Ile Leu Ser Glu Phe Tyr Ile Leu
   260 265 270
   Thr Ala Ala His Cys Leu Tyr Gln Ala Lys Arg Phe Lys Val Arg Val
   275 280 285
   Gly Asp Arg Asn Thr Glu Gln Glu Glu Gly Gly Glu Ala Val His Glu
   290 295 300
   Val Glu Val Val Ile Lys His Asn Arg Phe Thr Lys Glu Thr Tyr Asp
   305 310 315 320
   Phe Asp Ile Ala Val Leu Arg Leu Lys Thr Pro Ile Thr Phe Arg Met
   325 330 335
   Asn Val Ala Pro Ala Cys Leu Pro Glu Arg Asp Trp Ala Glu Ser Thr
   340 345 350
   Leu Met Thr Gln Lys Thr Gly Ile Val Ser Gly Phe Gly Arg Thr His
   355 360 365
   Glu Lys Gly Arg Gln Ser Thr Arg Leu Lys Met Leu Glu Val Pro Tyr
   370 375 380
   Val Asp Arg Asn Ser Cys Lys Leu Ser Ser Ser Phe Ile Ile Thr Gln
   385 390 395 400
   Asn Met Phe Cys Ala Gly Tyr Asp Thr Lys Gln Glu Asp Ala Cys Gln
   405 410 415
   Gly Asp Ser Gly Gly Pro His Val Thr Arg Phe Lys Asp Thr Tyr Phe
               420                 425                 430
   Val Thr Gly Ile Val Ser Trp Gly Glu Ser Cys Ala Arg Lys Gly Lys
           435                 440                 445
   Tyr Gly Ile Tyr Thr Lys Val Thr Ala Phe Leu Lys Trp Ile Asp Arg
       450                 455                 460
   Ser Met Lys Thr Arg Gly Leu Pro Lys Ala Lys Ser His Ala Pro Glu
   465                 470                 475                 480
   Val Ile Thr Ser Ser Pro Leu Lys  *
                   485